# EUROPEAN PATENT SPECIFICATION

(11) **EP 3 869 192 B1**
(45) Date of publication and mention of the grant of the patent: **13.05.2026**
(21) Application number: 19872428.8
(22) Date of filing: 21.10.2019
(51) Int. Cl.: G01N 30/06, G01N 30/04, G01N 33/48, G01N 33/487, G01N 33/68, B01L 3/00, G01N 1/28, G01N 33/50, G01N 30/72, G01N 30/88

(54) **METHOD FOR QUANTITATIVE DETECTION OF VARIOUS METABOLITES IN BIOLOGICAL SAMPLE**
VERFAHREN ZUR QUANTITATIVEN DETEKTION VERSCHIEDENER METABOLITEN IN BIOLOGISCHEN PROBEN
MÉTHODE DE DÉTECTION QUANTITATIVE DE DIVERS MÉTABOLITES DANS UN ÉCHANTILLON BIOLOGIQUE

(30) Priority: 19.10.2018 CN 201811223486
(43) Date of publication of application: 25.08.2021
(73) Proprietor: Human Metabolomics Institute, Inc., Shenzhen, Guangdong 518110 (CN)
(72) Inventor: JIA, Wei, Shenzhen, Guangdong 518110 (CN); XIE, Guoxiang, Shenzhen, Guangdong 518110 (CN); WANG, Lu, Shenzhen, Guangdong 518110 (CN)
(74) Representative: dompatent
(86) International application number: PCT/CN2019/112389
(87) International publication number: WO 2020/078478

(56) References cited:
- EP-A2- 1 875 401
- WO-A2-2017/070114
- CN-A- 107 870 214
- JP-A- 2015 087 199
- JP-A- H02 114 175
- HAN JUN ET AL: "Isotope-labeling derivatization with 3-nitrophenylhydrazine for LC/multiple-reaction monitoring-mass-spectrometry-based quantitation of carnitines in dried blood spots", ANALYTICA CHIMICA ACTA, ELSEVIER, AMSTERDAM, NL, vol. 1037, 5 February 2018 (2018-02-05), pages 177 - 187, XP085495406, ISSN: 0003-2670, DOI: 10.1016/J.ACA.2018.01.045
- HAN JUN ET AL: "An isotope-labeled chemical derivatization method for the quantitation of short-chain fatty acids in human feces by liquid chromatography-tandem mass spectrometry", ANALYTICA CHIMICA ACTA, vol. 854, 15 November 2014 (2014-11-15), AMSTERDAM, NL, pages 86 - 94, XP055541558, ISSN: 0003-2670, DOI: 10.1016/j.aca.2014.11.015
- SCH�TT HANS-FRIEDER ET AL: "A Validated, Fast Method for Quantification of Sterols and Gut Microbiome Derived 5[alpha]/[beta]-Stanols in Human Feces by Isotope Dilution LC-High-Resolution MS", ANALYTICAL CHEMISTRY, vol. 90, no. 14, 19 June 2018 (2018-06-19), US, pages 8487 - 8494, XP055930557, ISSN: 0003-2700, DOI: 10.1021/acs.analchem.8b01278
- HAN, JUN ET AL.: "An Isotope-Labeled Chemical Derivatization Method for the Quantitation of Short-Chain Fatty Acids in Human Feces by Liquid Chromatography-Tandem Mass Spectrometry", ANALYTICA CHIMICA ACTA, vol. 854, 15 November 2014 (2014-11-15), pages 86 - 94, XP055541558, DOI: 10.1016/j.aca.2014.11.015
- HAN, JUN ET AL.: "Quantitation of Low Molecular Weight Sugars by Chemical Derivatization- Liquid Chromatography/Multiple Reaction Monitoring/Mass Spectrometry", ELECTROPHORESIS, vol. 37, no. 13, 31 December 2016 (2016-12-31), pages 1851 - 1860, XP055807955
- ZENG, MINGFEI ET AL.: "Fast Quantification of Short Chain Fatty Acids and Ketone Bodies by Liquid Chromatography-Tandem Mass Spectrometry after Facile Derivatization Coupled with Liquid-Liquid Extraction", JOURNAL OF CHROMATOGRAPHY B, vol. 1083, 8 March 2018 (2018-03-08), pages 137 - 145, XP085371247, DOI: 10.1016/j.jchromb.2018.02.040

## Description

### BACKGROUND

### Technical Field

The present invention relates to the field of detection of biological samples, specifically relates to a quantitative determination method of multiple metabolic components such as amino acid, phenol, phenyl or benzyl derivative, indole, organic acid, fatty acid, sugar, and bile acid in a biological sample, and more specifically relates to a detection method of a biological sample by using chemical derivatization and tandem mass spectrometry and use of a a metabolic chip in the method.

### Related Art

Metabolomics involves unbiased analysis of all metabolites (metabolomes) in cells, body fluids and tissues. At present, with a metabolomics platform based on nuclear magnetic resonance (NMR) or mass spectrometry (MS), many small molecule (MW<1500) metabolites are detected, but only relative (non-absolute) concentrations of the metabolites in biological fluids (serum/plasma or urine) and tissues of subjects suffering from metabolic diseases are provided to determine that the concentrations are different from those of a control group. Due to high chemical diversity of the metabolomes, there is a great challenge to full-spectrum quantitative detection of these metabolites. Since a quantitative metabolomics platform for large-scale biological sample analysis is in deficiency, clinical practicality and application of metabolomics have not yet been realized.

Quantitative metabolomics is used for identifying and quantifying as many metabolites as possible in a biological sample. Compared with traditional targeted and non-targeted methods, quantitative metabolomics has many advantages, including lowest cross-platform variability, improved stability and maintenance of full-spectrum metabolic characteristics and more detailed information about the identity and concentration of specific metabolites.

With regard to quantification of metabolite concentration, reliable analytical data is a prerequisite for development of metabolic-based clinical trials or a thorough understanding of functions of organisms and biological systems in translational researches.

One of the technical challenges is that concentrations of metabolites are in a range of more than a dozen of magnitudes. For example, glucose in blood is in some compounds such as eicosanoids in a millimolar range and a femtomolar range. There are also different platform challenges in size and polarity differences of compounds. In order to overcome these challenges, gas chromatography-mass spectrometry (GC-MS) and liquid chromatography-mass spectrometry (LC-MS) have been applied to a maximum coverage. However, the difficulty in quantitative detection of multiple indexes at the same time has not yet been solved.

There are only a few major metabolic pathways, such as glycolysis, aerobic respiration, tricarboxylic acid (TCA) cycle, fatty acid oxidation (β-oxidation) and gluconeogenesis. Cells are used to transfer energy and maintain metabolic homeostasis, and due to defects in these key pathways in storage and disposal of major classes of molecules (such as amino acid, carbohydrate, and lipid), metabolic disorders are caused. Therefore, unique characteristics of metabolites in biological systems can be reflected in quantitative detection of these metabolites.

As important substances involved in physiological metabolism of the human body, amino acid, phenol, phenyl or benzyl derivative, indole, organic acid, fatty acid, sugar, and bile acid are maintained at a certain level in normal metabolism in the human body. Generally, the changes of concentrations of these substances indicate that there are abnormalities in metabolic pathways of the human body. Based on detection of concentrations of these substances and clinical manifestations, judgment of clinical diseases is facilitated.

Han Jun et al. (Isotope-labeling derivatization with 3-nitrophenyhydrazine for LC/multiple-reaction monitoring-mass-spectrometry-based quantitation of carnitines in dried blood spots: Analytica Chimica Acta, vol 1037, 5. February 2018, pages 177-187) and Han Jun et al. (An isotope-labeled chemical derivatization method for the quantitation of short-chain fatty acids in human feces by liquid chromatography-tandem mass spectrometry: Analytica Chimica Acta, Vol 854, 1 January 2015, pages 86-94) disclose a quantitative detection method of multiple metabolic components in a biological sample.

In view of the shortcomings of the prior art, an objective of the present invention is to provide a detection method for quantitatively detecting multiple components in a biological sample at the same time.

### SUMMARY

An objective of the present invention is to provide a quantitative detection method capable of quantitatively detecting multiple metabolic components in a biological sample at the same time and the use of a metabolic chip in the method. The multiple metabolic components include, but are not limited to, multiple amino acids, phenols, phenyl or benzyl derivatives, indoles, organic acids, fatty acids, sugars, and bile acids.

In the present invention, the quantitative detection method of multiple metabolic components in a biological sample is achieved by using the following solution: derivatization treatment is performed on the biological sample, and then the derivatized biological sample is detected by liquid chromatography-mass spectrometry; during derivatization treatment, 3-nitrophenylhydrazine is used as a derivatization reagent, and 1-(3-dimethylaminopropyl)-3-ethylcarbodiimide is used as a derivatization reaction catalyst; the biological sample is selected from urine, blood, cerebrospinal fluid, tissue, cell, saliva and fecal samples of a mammal; the multiple metabolic components in the biological sample are selected from one or more of amino acid, phenol, phenyl or benzyl derivative, indole, organic acid, fatty acid, sugar, and bile acid and have different magnitudes in content.

The detection method of the present invention includes the following steps:
a) collecting a biological sample;
b) extracting the biological sample with a mixed solvent of methanol, chloroform, and water, performing centrifugation, and taking a supernatant, namely a biological sample extract;
c) adding the same volume of a 3-nitrophenylhydrazine methanol solution and a 1-(3-dimethylaminopropyl)-3-ethylcarbodiimide pyridine solution into the biological sample extract obtained in b), and performing uniform vortex mixing and heating for derivatization, where the concentration of used 3-nitrophenylhydrazine is 100-320 mmol/L (in this patent application, "mM" represents "mmol/L"), the concentration of 1-(3-dimethylaminopropyl)-3-ethylcarbodiimide is 50-200 mmol/L, the reaction temperature is 20-60°C, and the reaction time is 10-120 minutes;
d) adding a carbon-13 labeled isotope internal standard solution obtained from the reaction of 3-nitrophenylhydrazine and 1-(3-dimethylaminopropyl)-3-ethylcarbodiimide into the derivatized biological sample extract obtained in c); and
e) adding a methanol-water mixed solution into a sample in d) for dilution, and determining amino acid, phenol, phenyl or benzyl derivative, indole, organic acid, fatty acid, sugar, and bile acid by liquid chromatography-mass spectrometry.

Preferably, in step c), the concentration of used 3-nitrophenylhydrazine is 150-220 mM, the concentration of 1-(3-dimethylaminopropyl)-3-ethylcarbodiimide is 80-120 mM, the reaction temperature is 20-40°C, and the reaction time is 30-60 minutes.

Preferably, the volume ratio of methanol to water in step e) is 1:1.

When the biological sample is urine, blood, saliva or cerebrospinal fluid, a treatment method of the biological sample in step b) preferably includes: taking an appropriate amount of the biological sample, extracting the biological sample with a mixed solvent of cold methanol, chloroform and water at a volume ratio of 3:1:1, shaking the mixture for a few seconds, performing centrifugation on the sample at a rotation speed of 10000-20000 rpm at a low temperature for 5-15 minutes, and transferring a supernatant into an autosampler glass vial for subsequent derivatization determination.

When the biological sample is a fecal sample, a treatment method of the biological sample in step b) preferably includes: freeze-drying the fecal sample; uniformly mixing an appropriate amount of the freeze-dried fecal sample and an appropriate amount of a mixed solvent of cold methanol, chloroform and water at a volume ratio of 3:1:1, performing centrifugation on the sample at a rotation speed of 10000-20000 rpm at a low temperature for 15-30 minutes, and transferring a supernatant into an autosampler glass vial for subsequent derivatization treatment.

When the biological sample is a tissue or cell sample, a treatment method of the biological sample in step b) preferably includes: adding an appropriate amount of a mixed solvent of cold methanol, chloroform, and water at a volume ratio of 3:1:1 into the sample for homogenizing, performing centrifugation on the sample at a rotation speed of 10000-20000 rpm at 4°C for 15-30 minutes, and transferring a supernatant into an autosampler glass vial for subsequent derivatization treatment.

According to the detection method provided in the present invention, 3-nitrophenylhydrazine is used to undergo a derivatization reaction with amino acid, phenol, phenyl or benzyl derivative, indole, organic acid, fatty acid, sugar, and bile acid in the sample in the presence of 1-(3-dimethylaminopropyl)-3-ethylcarbodiimide to produce corresponding derivatives, the detection sensitivity is improved, the detection difficulty is reduced, quantitative detection of multiple substances of different magnitudes in the biological sample can be achieved, defects in the prior art are overcome, and a high-throughput quantitative detection effect is achieved. In addition, in the present invention, a commonly labeled derivatization reagent and a carbon-13 labeled derivatization reagent are used to undergo a reaction with the standard product and the sample solution so that chromatographic behaviors, ionization efficiency of mass spectrometry and matrix effect can be completely consistent, and thus systematic errors are avoided.

The present invention provides a quantitative detection method of multiple metabolic components of different magnitudes in a biological sample. As understood by a person of ordinary skill, the content of index components in the sample can be calculated by drawing a standard curve in the present invention. On this basis, the applicant further provides a metabolic chip used in the detection method. The metabolic chip includes

The present invention further relates to the use of a metabolic chip in the detection method. The metabolic chip is a device for efficient quantitative detection of multiple metabolic components by using the detection method of the present invention, including a chip carrier, a filter device and dry solid powder of a standard product and a quality control product. The chip carrier is a microtiter plate, and the microtiter plate may be a commercially available 48-well plate, a 96-well plate, and a 384-well microtiter plate suitable for liquid chromatography determination. Each well of the microtiter plate is provided with an independent filter device, and the filter device is a filter membrane made of polyvinylidene fluoride, cellulose acetate, or nylon with a pore size of 0.20-0.45 micron (µm). Each well of the microtiter plate is divided into upper and lower parts by the filter device.

The dry solid powder of the standard product and the quality control product is powder obtained by dehydrating or freeze-drying solutions of the standard product and the quality control product and is placed on the filter device in each well of the microtiter plate. As understood by a person of ordinary skill in the art, when the powder of the standard product is prepared, different standard product solutions are prepared first according to required standard product concentration gradients based on the drawn standard curve, then dehydrating or freeze-drying is performed to obtain the powder, and the powder is placed on the corresponding filter devices in the wells of the metabolic chip. As understood by a person of ordinary skill in the art, the quality control product is prepared into a corresponding solution and then dehydrated or freeze-dried to obtain the powder, and the powder is placed on the filter device in each well of the metabolic chip.

The standard product is selected from one or more of amino acid, phenol, phenyl or benzyl derivative, indole, organic acid, fatty acid, sugar, and bile acid standard products. The quality control product is selected from one or more of amino acid, phenol, phenyl or benzyl derivative, indole, organic acid, fatty acid, sugar, and bile acid standard products corresponding to the standard products above.

Specifically, the standard product and the quality control product may be selected from the following components: fructose 1,6-diphosphate, 10Z-nonadecenoic acid, trans-11-octadecenoic acid, cis-11-octadecenoic acid, 12-dehydrocholic acid, 12-hydroxystearic acid, 12-ketolithocholic acid, 1-methylhistidine, 2,2-dimethylsuccinic acid, 2,3-diaminopropionic acid, glucoside 24-chenodeoxycholic acid, 2-butenoic acid, 2-oxoadipic acid, 2-methyl-4-pentenoic acid, 2-methyl-β-alanine, 2-methylbutyric acid, 2-methylhexanoic acid, 2-methylglutaric acid, 2-methylglutamic acid, 2-furoic acid, 2-hydroxy-2-methylbutyric acid, 2-hydroxy-3-methylbutyric acid, 2-hydroxybutyric acid, 2-hydroxycaproic acid, 2-hydroxycinnamic acid, 2-hydroxyphenylacetic acid, 2-hydroxyhippuric acid, 2-phenylpropionic acid, 2-phenylglycine, 3-(3-hydroxyphenyl)-3-hydroxypropionic acid, 3-(4-hydroxyphenyl)lactic acid, 3,4-dihydroxymandelic acid, 3,4-dihydroxyphenylpropionic acid, 3,4 -dehydro-DL-proline, 3,5-diiodo-L-thyroxine, 3β-cholic acid, 3-pyridineacetic acid, 3-indoleacrylic acid, 3-indolepropionic acid, 3-indoleacetamide, 3-oxyalanine, 3-aminosalicylic acid, 3-chloro-L-tyrosine, 3-methyl-2-oxobutyric acid, 3-methyl-2-oxovaleric acid, 3-methylindole, 3-methyladipic acid, 3-methylglutamic acid, 3-nitrotyrosine, sulfate 3-taurolithocholic acid, sulfate 3-lithocholic acid, 3-hydroxy-2-aminobenzoic acid, 3-hydroxybutyric acid, 3-hydroxypropionic acid, 3-hydroxyisovaleric acid, 3-hydroxyphenylacetic acid, 3-hydroxyhippuric acid, 3-dehydrocholic acid, 3-phenylbutyric acid, 4-methyl-2-oxovaleric acid, 4-methylhexanoic acid, 4-methoxyphenylacetic acid, 4-hydroxy-3-methoxymandelic acid, 4-hydroxycinnamic acid, 4-hydroxybenzoic acid, 4-hydroxyhippuric acid, 4-hydroxyphenyllactic acid, 4-hydroxyphenylpyruvic acid, 4-phenylbutyric acid, 5-aminolevulinic acid, 5-hydroxytryptophan, 5-hydroxytryptamine, 5-hydroxylysine, 6,7-diketolithocholic acid, 6-phosphogluconic acid, 6-ketolithocholic acid, 7,12-diketolithocholic acid, 7-ketolithocholic acid, 7-ketodeoxycholic acid, 9,11-conjugated linoleic acid, D-2-hydroxyglutaric acid, D-galactose, D-xylose, D-xylulose, D-fructose, D-ribose, D-ribose-5-phosphate, D-ribulose, D-ribulose-5-phosphate, D-mannose, D-glucose, D-maltose, L-2-aminobutyric acid, L-3-phenyllactic acid, L-alanine, L-serine, L-acetylcarnitine, L-lactic acid, L-allothreonine, L-cysteine, L-homoserine, L-homocysteine, L-homocitrulline, L-pipecolic acid, L-aspartic acid, L-asparagine, L-sorbose, L-lignic acid, L-norleucine, L-kynurenine, L-thyronine, L-arginine, L-histidine, L-valine, L-cystine, L-cystathionine, L-proline, L-tryptophan, L-threonine, L-phenylalanine, L-malic acid, L-methionine, L-glutamine, L-glutamic acid, L-lysine, L-tyrosine, L-arabinose, N-(3-phenylpropionyl)glycine, N-acetyl-L-alanine, N-acetyl-L-aspartic acid, N-acetyl-L-phenylalanine, N-acetyl-L-methionine, N-acetyl-L-tyrosine, N-acetylserine, N-acetyl-D-glucosamine, N-acetyl-L-phenylalanine, N-acetylmannosamine, N-acetylneuraminic acid, N-acetylhistidine, N-acetylhydroxytryptamine, N-acetyltryptophan, N-acetylglutamine, N-acetyllysine, N-acetylornithine, N-methylnicotinamide, N-phenylacetyl-glutamine, N-phenylacetylphenylalanine, S-adenosine homocysteine, α-D-glucose, α-lactose, α-linolenic acid, α-hydroxyisobutyric acid, α-ketoglutaric acid, α-muricholic acid, β-D-trehalose, β-alanine, β-ursocholic acid, β-muricholic acid, γ-L-glutamyl-L-alanine, γ-linolenic acid, γ-aminobutyric acid, ω-muricholic acid, butyric acid, trimethylamine nitroxides, adenosine triphosphate, malonic acid, propionic acid, acetoacetic acid, acetylcysteine, acetylglycine, acetylomithine, acetic acid, guanidine acetate, glycolic acid, lactulose, lactoylglutathione, heneicosanoic acid, cis-12-heneicosenoic acid, heptacosanoic acid, tricosanoic acid, cis-14-tricosenoic acid, docosanoic acid, docosatrienoic acid, cis-13,16-docosadienoic acid, docosapentaenoic acid, docosahexaenoic acid, docosatetraenoic acid, trans-13-docosaenoic acid, cis-13-docosaenoic acid, pentacosanoic acid, octacosanoic acid, hexacosanoic acid, tetracosanoic acid, eicosanoic acid, eicosatrienoic acid, eicosadienoic acid, eicosapentaenoic acid, trans-11-eicosenoic acid, cis-11-eicosenoic acid, cis-5-eicosenoic acid, cis-8-eicosenoic acid, dimethylglycine, adenosine diphosphate, linoleic acid, leucine, alloisoleucine, allolithocholic acid, allocholic acid, undecenoic acid, heptadecanoic acid, tridecanoic acid, nonadecanoic acid, nonadecadienoic acid, pentadecanoic acid, galactonic acid, galactitol, mecysteine, protocatechuic acid, apocholic acid, dehydrolithocholic acid, nordeoxycholic acid, trans-9-tetradecenoic acid, trans-aconitic acid, trans-4-hydroxyproline, trans-9-heptadecenoic acid, trans-9-pentadecenoic acid, trans-9-hexadecenoic acid, trans-linolenic acid, trans-cinnamic acid, elaidic acid, homocysteine, pyrrole-2-carboxylic acid, picolinic acid, indole, indole-3-methyl acetate, indole-3-carboxylic acid, indoleacetic acid, purine, azelaic acid, nonanoic acid, dopa, dopamine, melibiose, fumaric acid, acetaminophen, p-aminohippuric acid, p-cresol sulfate, symmetrical dimethylarginine, p-hydroxymandelic acid, p-hydroxyphenylacetic acid, homogentisic acid, adipic acid, pimelic acid, isobutyric acid, isoleucine, isovaleric acid, citric acid, isoursodeoxycholic acid, isohyodeoxycholic acid, isolithocholic acid, isocholic acid, isodeoxycholic acid, glutaric acid, glutaconic acid, valeric acid, mandelic acid, lauric acid, fructose-6-phosphate, citraconic acid, citric acid, citramalic acid, ribonolactone, ribonic acid, raffinose, palmitoleic acid, palmitic acid, norvaline, n-hydroxyphenylacetic acid, oxidized glutathione, aminoadipic acid, aminocaproic acid, salicyluric acid, oleic acid, trehalose, nicotinic acid, pyroglutamic acid, ursocholic acid, ursodeoxycholic acid, tauro-α-muricholic acid, tauro-b-muricholic acid, tauro-w-muricholic acid, tauroursodeoxycholic acid, taurohyocholic acid, taurohyodeoxycholic acid, taurolithocholic acid, taurocholic acid, taurodehydrocholic acid, taurochenodeoxycholic acid, hyocholic acid, hyodeoxycholic acid, succinylacetone, succinic acid, citrulline, glycodehydrocholic acid, glycolithocholic acid, glycodeoxycholic acid, glycine, glycochenodeoxycholic acid, glyceraldehyde, glyceraldehyde-3-phosphate, choline glycerophosphate, glycoursodeoxycholic acid, glycohyocholic acid, glycohyodeoxycholic acid, glycocholic acid, glyproline, glycyl-L-leucine, mannose-6-phosphoric acid, mannitol, methylmalonic acid, methylsuccinic acid, formic acid, capric acid, lithocholic acid, selenomethionine, thiamine, glycyllithocholic sulfate, stearic acid, liothyronine, dihydroxyacetone phosphate, phosphoribosyl pyrophosphate, creatine phosphate, hydroxypyruvic acid, glycine hydroxyphenylacetate, cinnamic acid, sarcosine, carnosine, creatine, inositol, epinephrine, choline, cholic acid, dehydrocholic acid, demethylcholic acid, adenosine monophosphate, arachidonic acid, phenylpyruvic acid, phenethylamine, phenylpropionic acid, phenyllactic acid, benzamide, benzoic acid, oxalic acid, shikimic acid, glucaric acid, glucose-6-phosphate, glucose lactone, sebacic acid, ricinoleic acid, sucrose, methionine sulfoxide, itaconic acid, melatonin, glutathione, myristic acid, erythronic acid, erythrose, suberic acid, caprylic acid, phthalic acid, tartaric acid, tyramine, quinic acid, m-aminobenzoic acid, asymmetric dimethylarginine, tannic acid, cis-10,12-octadecadienoic acid, cis-12,15-heneicosadienoic acid, cis-12-tridecenoic acid, cis-15-tetracosenic acid, cis-2-hydroxycinnamic acid, cis-9-tetradecenoic acid, cis-10-heptadecenoic acid, cis-11-dodecenoic acid, cis-4-hydroxyproline, cis-5-dodecenoic acid, cis-7-hexadecenoic acid, cis-9-heptadecenoic acid, cis-aconitic acid, vanillic acid, hippuric acid, maleic acid, homovanillic acid, ornithine, guanosine monophosphate, guanosine triphosphate, anserine, chenodeoxycholic acid, maltotriose, maltitol, rhamnose and murideoxycholic acid.

The use of the metabolic chip in the present invention includes the following steps:
1. collecting a biological sample;
2. according to the sample type, preparing a corresponding biological sample extract by using the corresponding method;
3. adding the prepared biological sample extract into each well of the metabolic chip in an equal amount, adding the same volume of a 3-nitrophenylhydrazine methanol solution and a 1-(3-dimethylaminopropyl)-3-ethylcarbodiimide pyridine solution into each well, and performing uniform vortex mixing and heating for derivatization, where the concentration of used 3-nitrophenylhydrazine is 100-320 nM, the concentration of 1-(3-dimethylaminopropyl)-3-ethylcarbodiimide is 50-200 nM, the reaction temperature is 20-60°C, and the reaction time is 10-120 minutes;
4. adding a carbon-13 labeled isotope internal standard solution obtained from the reaction of 3-nitrophenylhydrazine and 1-(3-dimethylaminopropyl)-3-ethylcarbodiimide into the derivatized biological sample extract obtained in step 3;
5. adding a methanol-water mixed solution into each well in the metabolic chip in step 4 for dilution, placing the metabolic chip in a tandem mass spectrometer for liquid chromatography-mass spectrometry for determination of amino acid, phenol, phenyl or benzyl derivative, indole, organic acid, fatty acid, sugar, and bile acid by liquid chromatography-mass spectrometry, and calculating concentrations of target metabolites in the sample based on results.

As understood by a person of ordinary skill in the art, the content of each target detection substance can be calculated based on detection results and the standard curve. The detection results obtained by the metabolic chip can also be obtained by calculation with a metabolite batch quantification software developed by Shenzhen Huiyun Biotechnology Co., Ltd. to quickly obtain the content of each target component. By combining the calculation software with the metabolic chip of the present invention, the work efficiency is greatly improved.

### BRIEF DESCRIPTION OF THE DRAWINGS

The accompanying drawings are chromatograms of typical amino acid, organic acid, fatty acid, sugar, and bile acid detected in blood samples in embodiments. It can be seen that multiple target detection components can be effectively separated and detected, and thus high-throughput quantitative detection is achieved.

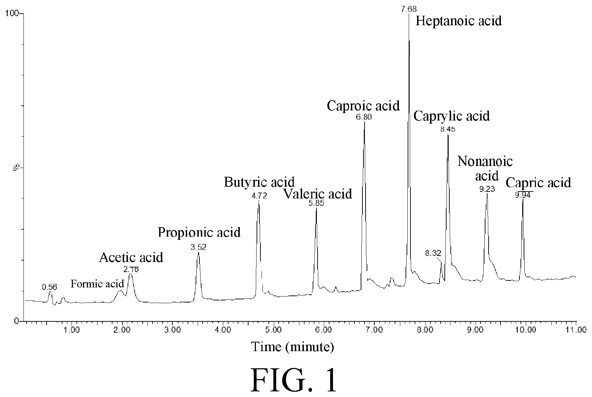

FIG. 1 is a chromatogram of typical short-chain fatty acid;

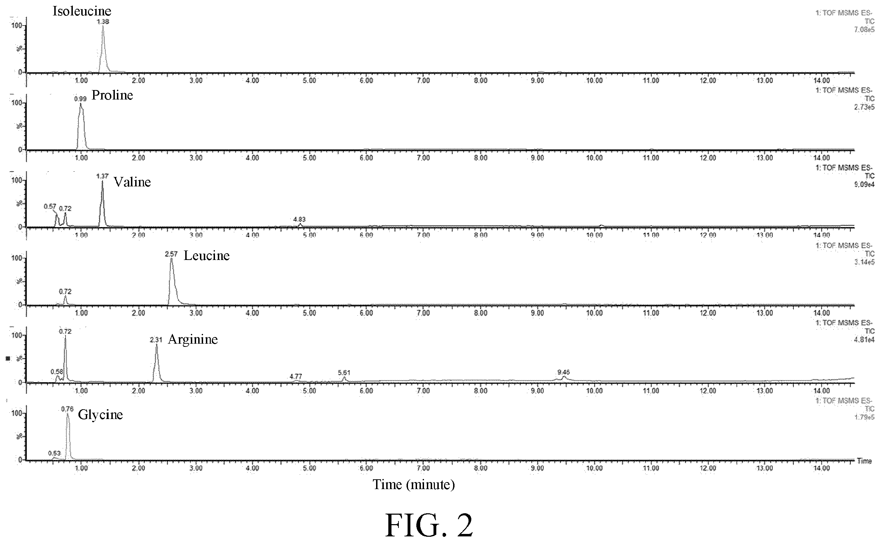

FIG. 2 is a chromatogram of typical amino acid;

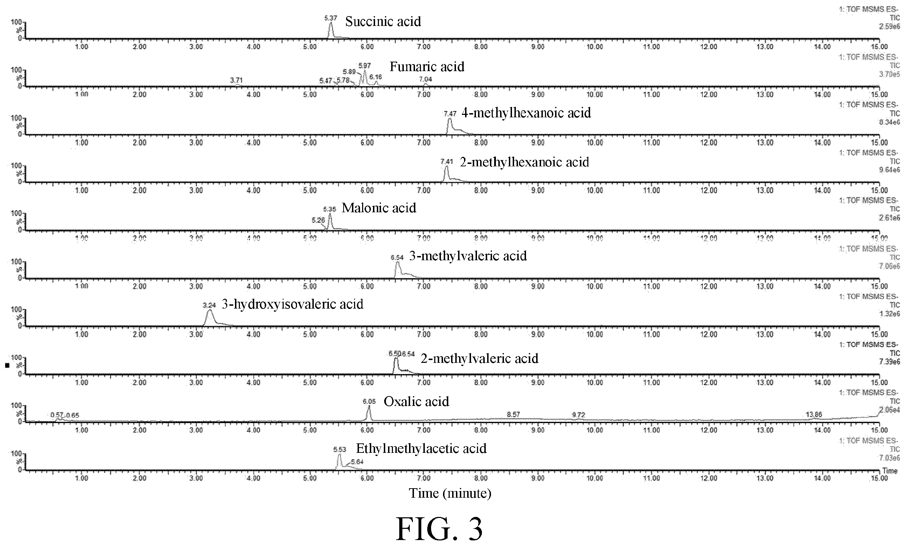

FIG. 3 is a chromatogram of typical organic acid;

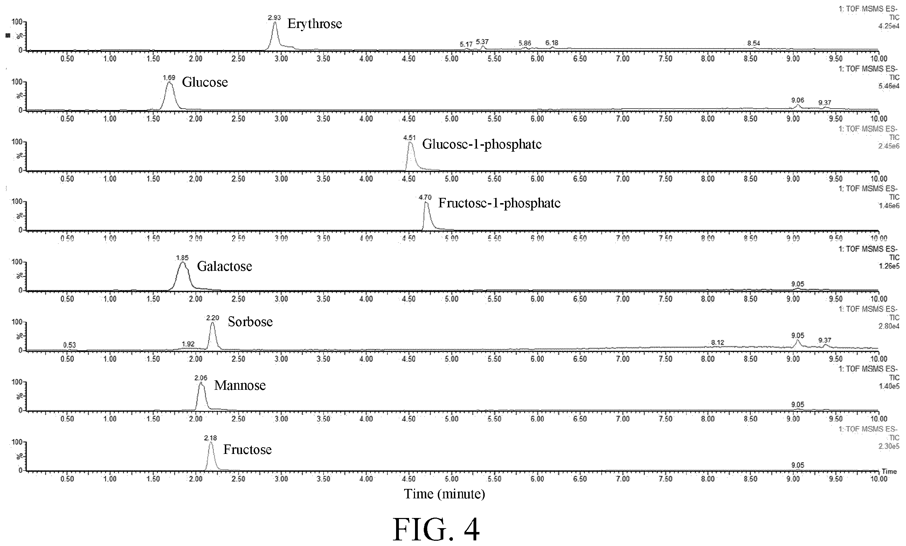

FIG. 4 is a chromatogram of typical sugar;

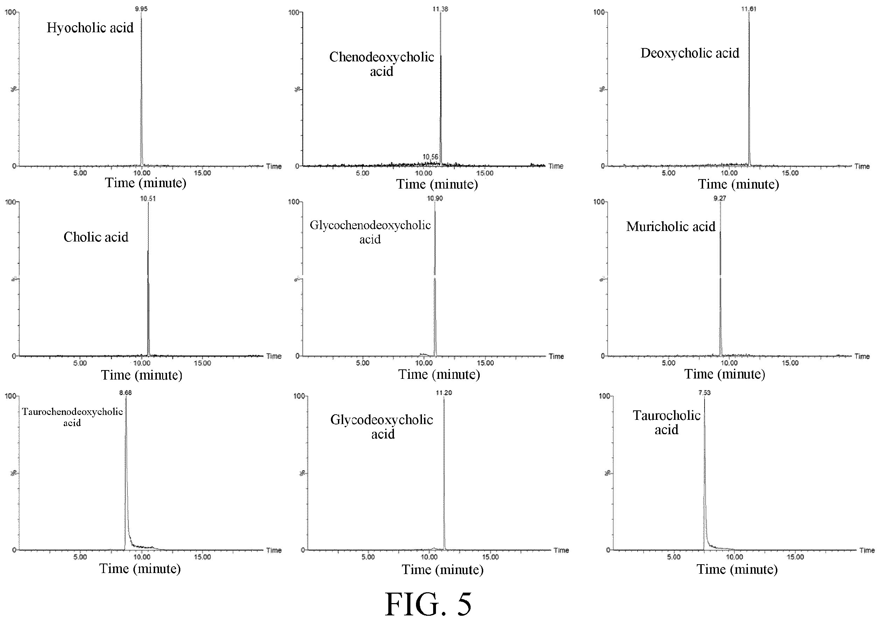

FIG. 5 is a chromatogram of typical bile acid.

### DETAILED DESCRIPTION

A metabolic chip is used to detect multiple index components in 10 human blood and fecal samples.

### 1. Instrument

Liquid chromatography-tandem mass spectrometer (LC-MS/MS) equipped with an electrospray ionization source (ESI).

### 2. Sample preparation

Serum sample: A venous whole blood sample is collected, placed in an anticoagulation tube, then immediately shaken up and down for uniform mixing 5-6 times, and centrifugated within 30 minutes to separate plasma. The sample is placed in a centrifuge tube, extracted with a mixed solvent of cold methanol, chloroform and water at a volume ratio of 3:1:1, shaken for a few seconds and then centrifugated at a rotation speed of 10000-20000 rpm at 4°C for 5-15 minutes to obtain a supernatant. The supernatant is transferred into an autosampler glass vial. All water-containing serum or urine sample extracts are used for subsequent derivatization treatment.

Fecal sample: A fecal sample is freeze-dried. An appropriate amount of the freeze-dried fecal sample and an appropriate amount of a mixed solvent of cold methanol, chloroform, and water at a volume ratio of 3:1:1 are homogenized. The sample is centrifugated at a rotation speed of 10000-20000 rpm at 4°C for 15-30 minutes to obtain a supernatant. The supernatant is transferred into an autosampler glass vial for subsequent derivatization treatment.

### 3. Reagent preparation

Preparation of standard product solution: Standard products, including amino acid, phenol, phenyl or benzyl derivative, indole, organic acid, fatty acid, sugar, and bile acid, of metabolites in the description are taken, fully dissolved in methanol and uniformly mixed to prepare a 1 mg/ml solution, namely a concentrated stock solution which is prepared into a series of concentrations of solutions to draw a standard curve.

Preparation of quality control product solution: A corresponding quality control product is taken, fully dissolved in methanol and uniformly mixed to prepare a 1 mg/ml solution, namely a concentrated stock solution, and then the solution is diluted to a certain concentration and reacts with carbon-13 labeled 3-nitrophenylhydrazine and 1-(3-dimethylaminopropyl)-3-ethylcarbodiimide to obtain the quality control product solution.

Preparation of derivatization reagent (3-nitrophenylhydrazine): A derivatization reagent is uniformly mixed with a 75% methanol aqueous solution to prepare a 200 mM solution, and the solution is sealed and stored at 4°C for later use.

Preparation of derivatization reaction catalyst (1-(3-dimethylaminopropyl)-3-ethylcarbodiimide): A reaction catalyst is prepared into a 120 millimolar solution with pyridine, and the solution is sealed and stored at 4°C for later use.

### 4. Inspection method

5 µL of a treated biological sample is taken, 20 µl of a derivatization reagent and 20 µl of a derivatization catalyst are added for reaction at 30°C for 60 minutes, a methanol-water mixed solution is added into a reaction solution for dilution, centrifugation is performed at 13200 rpm for 15 minutes, and 5 µL of a supernatant is taken and introduced for LC-MS/MS analysis.

### Mass spectrometry conditions:

Ion source: Multi-reaction detection conditions for detection of multiple substances by tandem mass spectrometry are shown in Table 1. A negative ion scanning mode (ESI-) is adopted for an electrospray ion source, and specific conditions are as follows: The capillary voltage is 1.2 kV, the cone voltage is 55 V, the extraction cone voltage is 4 V, the ion source temperature is 150°C, the desolvent gas temperature is 550°C, the reverse cone gas flow is 50 L/h, the desolvent gas is 650 L/h, the resolution of a low mass zone is 4.7, the resolution of a high mass zone is 15, and a multi-reaction detection mode is used to collect data.

Gradient elution conditions: A UPLC BEH C18 chromatographic column (100 mm * 2.1 mm, 1.7 µm) is used; the column temperature is 40°C; a mobile phase A includes water (0.1% formic acid), and a mobile phase B includes acetonitrile (0.1% formic acid) and isopropanol at a ratio of (1-2):1; the flow rate is 0.4 mL/min; the injection volume is 5 microliters; and gradient elution conditions: 0-1 min (5% B), 1-5 min ( 5-30% B), 5-9 min (30-50% B), 9-12 min (50-75% B), 12-15 min (75-95% B), 15-16 min (95-100% B), 16-18 min (100% B), 18-20 min (5% B).

### 5. Determination results

Concentrations of substances in 10 human blood and fecal samples are detected by using the detection method of the present invention (see Table 1 and Table 2 respectively). It can be seen that by using the method of the present invention to detect a single sample, multiple substances of different magnitudes and different properties can be quantitatively detected at one time.

**Table 1 Determination results of concentrations of substances in blood of normal people**

| Determined target metabolites | Concentration value | Concentration unit |
|---|---|---|
| 2-methylvaleric acid | 120.32±5.18 | µg/mL |
| 2-hydroxybutyric acid | 2.32±0.44 | µg/mL |
| 3-(3-hydroxyphenyl)-3-hydroxypropionic acid | 10.31±0.19 | µg/mL |
| 3-hydroxyphenylacetic acid | 6.07±6.12 | µg/mL |
| 3-indoleacetonitrile | 2.51±0.15 | µg/mL |
| 3-methyl-2-oxovaleric acid | 4.37±5.08 | µg/mL |
| 4-methylhexanoic acid | 2.96±0.07 | µg/mL |
| Linolenic acid | 97.19±33.87 | µg/mL |
| Arachidonic acid | 5.54±0.45 | µg/mL |
| Arachidonic acid | 5.62±2.4 | µg/mL |
| Docosanoic acid | 8.96±0.27 | µg/mL |
| β-alanine | 10.37±0.44 | µg/mL |
| Capric acid | 1.22±0.19 | µg/mL |
| Caprylic acid | 1.39±0.5 | µg/mL |
| Citric acid | 7.03±1.56 | µg/mL |
| Docosahexaenoic acid | 15.1±4.36 | µg/mL |
| Docosapentaenoic acid n6 | 15.31±4.16 | µg/mL |
| Docosatrienoic acid | 8.76±0.95 | µg/mL |
| Dodecanoic acid | 0.91±0.16 | µg/mL |
| Dopamine | 23.53±3.93 | µg/mL |
| Eicosenoic acid | 11.18±3.38 | µg/mL |
| Erucic acid | 15.25±11.34 | µg/mL |
| γ-aminobutyric acid | 14.68±2.7 | µg/mL |
| Glutathione | 6.7±1.62 | µg/mL |
| Glycolic acid | 159.46±56.76 | µg/mL |
| Heptadecanoic acid | 5.78±6.5 | µg/mL |
| L-α-aminobutyric acid | 2.41±0.48 | µg/mL |
| L-asparagine | 12.32±0.57 | µg/mL |
| L-aspartic acid | 3.8±0.64 | µg/mL |
| L-glutamic acid | 8.93±0.53 | µg/mL |
| L-histidine | 16.76±1.54 | µg/mL |
| L-homoserine | 12.53±7.81 | µg/mL |
| L-isoleucine | 5.9±2.18 | µg/mL |
| L-leucine | 5.88±3.97 | µg/mL |
| L-lysine | 33.73±4.42 | µg/mL |
| L-methionine | 6.78±0.73 | µg/mL |
| L-norleucine | 3.33±1.25 | µg/mL |
| L-phenylalanine | 8.57±0.84 | µg/mL |
| L-proline | 11.32±5.94 | µg/mL |
| L-serine | 9.78±0.68 | µg/mL |
| L-tryptophan | 30.49±2.93 | µg/mL |
| L-tyrosine | 20.02±4.37 | µg/mL |
| L-valine | 28.87±7.87 | µg/mL |
| Linoleic acid | 162.6±57.21 | µg/mL |
| Methylsuccinic acid | 34.76±29.31 | µg/mL |
| Myristic acid | 2.91±0.52 | µg/mL |
| Myristic acid | 3.39±0.51 | µg/mL |
| N-acetyltryptophan | 29.5±1.24 | µg/mL |
| Nervonic acid | 82.71±4.32 | µg/mL |
| Dodecanoic acid | 5.9±2.13 | µg/mL |
| Norvaline | 0.86±0.01 | µg/mL |
| Omithine | 22.56±5.54 | µg/mL |
| Carbonyladipic acid | 11.55±2.52 | µg/mL |
| Oxoglutaric acid | 21.89±5.22 | µg/mL |
| Palmitic acid | 87.29±30.74 | µg/mL |
| Palmitoleic acid | 86.36±30.14 | µg/mL |
| Pentadecanoic acid | 1.81±0.12 | µg/mL |
| Pimelic acid | 4.47±1.23 | µg/mL |
| Propionic acid | 0.16±0.03 | µg/mL |
| Putrescine | 19.96±1.32 | µg/mL |
| Pyroglutamic acid | 5.59±2 | µg/mL |
| Stearic acid | 38.06±14.04 | µg/mL |
| Succinic acid | 36.45±12.11 | µg/mL |
| Cis-aconitic acid | 2.66±0.11 | µg/mL |
| P-hydroxyphenylacetic acid | 2.51±0.15 | µg/mL |
| Palmitoleic acid | 19.32±11.42 | uM |
| Nervonic acid | 0.25±0.06 | uM |
| Cholic acid | 67.57±38.23 | nM |
| Chenodeoxycholic acid | 383±559.28 | nM |
| Deoxycholic acid | 241.97±197.98 | nM |
| Fructose | 5.23±1.18 | uM |
| Glucose | 3.29±0.66 | mM |
| Dohomo-g-linoleic acid | 2.51±1.01 | uM |
| Mannose | 34.87±9.22 | uM |
| Glycocholic acid | 314.89±345.38 | nM |
| Glycochenodeoxycholic acid | 750.99±574.2 | nM |
| Glycohyocholic acid | 16.31±10.67 | nM |
| Glycolithocholic acid | 12.41±10.41 | nM |
| Glycoursocholic acid | 123.97±124.56 | nM |
| Hyocholic acid | 26.09±11.26 | nM |
| Lithocholic acid | 12.61±4.68 | nM |
| Oleic acid | 239.05±84.26 | uM |
| Palmitoleic acid | 19.32±11.42 | uM |
| Taurocholic acid | 88.59±66.4 | nM |
| Taurodeoxycholic acid | 68.66±47.85 | nM |
| Teracosanoic acid | 2.28±0.89 | uM |
| Taurohyocholic acid | 4.06±4.7 | nM |
| Tauroursocholic acid | 26.16±0.81 | nM |
| Ursocholic acid | 69.29±44.3 | nM |
| g-linoleic acid | 20.47±8.36 | uM |

**Table 2 Determination results of concentrations of substances in feces of normal people**

| Detected target metabolites | Concentration value | Concentration unit |
|---|---|---|
| (1)-2-methylvaleric acid | 0.1±0.19 | µg/mL |
| 1H-indole-3-acetamide | 0.54±0.07 | µg/mL |
| 2-hydroxybutyric acid | 29.5±7.89 | ng/mL |
| 3-(3-hydroxyphenyl)-3-hydroxypropionic acid | 0.19±0.11 | µg/mL |
| 3-hydroxybutyric acid | 0.3±0.29 | µg/mL |
| 3-hydroxyphenylacetic acid | 0.39±0.36 | µg/mL |
| 3-indoleacetonitrile | 0.17±0.11 | µg/mL |
| 3-isopropionic acid | 0.32±0.09 | µg/mL |
| 3-methyl-2-oxovaleric acid | 0.5±0.2 | ng/mL |
| 3-methylvaleric acid | 0.2±0.3 | ng/mL |
| 4-hydroxybenzoic acid | 0.42±0.55 | µg/mL |
| 4-hydroxycinnamic acid | 0.25±0.07 | µg/mL |
| 4-methylhexanoic acid | 0.7±0.6 | ng/mL |
| 5-dodecenoic acid | 0.84±2.1 | µg/mL |
| Adipic acid | 88.6±10.6 | ng/mL |
| α-linolenic acid | 8.07±9.95 | µg/mL |
| Aminoadipic acid | 0.14±0.04 | µg/mL |
| Arachidonic acid | 3.83±2.82 | µg/mL |
| Arachidonic acid | 0.97±0.88 | µg/mL |
| Docosanoic acid | 0.33±0.32 | µg/mL |
| β-alanine | 0.41±0.12 | µg/mL |
| Butyric acid | 0.88±0.69 | µg/mL |
| Capric acid | 0.13±0.22 | µg/mL |
| Caproic acid | 0.53±0.53 | µg/mL |
| Caprylic acid | 0.37±0.55 | µg/mL |
| Citraconic acid | 45.8±4.97 | ng/mL |
| 2-methylmalic acid | 0.18±0.07 | µg/mL |
| Citric acid | 0.34±0.32 | µg/mL |
| D-2-hydroxyglutaric acid | 0.14±0.06 | µg/mL |
| Docosahexaenoic acid | 0.43±0.3 | µg/mL |
| Docosapentaenoic acid n6 | 0.45±0.41 | µg/mL |
| Docosatrienoic acid | 0.16±0.02 | µg/mL |
| Dodecanoic acid | 1.07±2.33 | µg/mL |
| Eicosenoic acid | 8.91±6.15 | µg/mL |
| Erucic acid | 0.41±0.36 | µg/mL |
| Ethylmethylacetic acid | 0.73±0.38 | µg/mL |
| Fumaric acid | 0.12±0.04 | µg/mL |
| γ-aminobutyric acid | 0.81±0.37 | µg/mL |
| Glutaric acid | 0.32±0.27 | µg/mL |
| Glutathione | 3.33±5.08 | µg/mL |
| Glyceric acid | 4.11±2.07 | µg/mL |
| Glycolic acid | 0.27±0.21 | µg/mL |
| Heptadecanoic acid | 1.06±1.15 | µg/mL |
| Heptanoic acid | 0.12±0.15 | µg/mL |
| Homocysteine | 1.1±1.01 | µg/mL |
| Hydrocinnamic acid | 0.36±0.21 | µg/mL |
| Hydroxyphenyllactic acid | 0.48±0.27 | µg/mL |
| Hydroxypropionic acid | 0.17±0.2 | µg/mL |
| Indole | 0.4±0.34 | µg/mL |
| Indoleacetic acid | 0.29±0.02 | µg/mL |
| Isocitric acid | 89.5±5.67 | ng/mL |
| Itaconic acid | 92.4±15.4 | ng/mL |
| L-α-aminobutyric acid | 74.7±16.7 | ng/mL |
| L-asparagine | 0.88±0.59 | µg/mL |
| L-aspartic acid | 2.44±1.03 | µg/mL |
| L-glutamic acid | 10.35±8.3 | µg/mL |
| L-histidine | 0.39±0.07 | µg/mL |
| L-homoserine | 0.45±0.42 | µg/mL |
| L-isoleucine | 0.23±0.11 | µg/mL |
| L-leucine | 0.25±0.42 | µg/mL |
| L-lysine | 2.29±0.65 | µg/mL |
| L-methionine | 1.54±0.91 | µg/mL |
| L-norleucine | 0.13±0.25 | µg/mL |
| L-phenylalanine | 0.85±0.91 | µg/mL |
| L-proline | 0.4±0.21 | µg/mL |
| L-serine | 0.42±0.31 | µg/mL |
| L-tryptophan | 0.62±0.16 | µg/mL |
| L-tyrosine | 2.19±1.06 | µg/mL |
| L-valine | 1.05±0.6 | µg/mL |
| Linoleic acid | 0.012±0.05 | mg/mL |
| Malic acid | 0.38±0.21 | µg/mL |
| Malonic acid | 0.12±0.06 | µg/mL |
| Methylsuccinic acid | 0.13±0.02 | µg/mL |
| Myristic acid | 0.54±0.54 | µg/mL |
| Myristic acid | 0.53±0.53 | µg/mL |
| N-acetyl | 0.61±0.07 | µg/mL |
| Nervonic acid | 1.33±0.9 | µg/mL |
| Nicotinic acid | 0.31±0.09 | µg/mL |
| Dodecanoic acid | 0.21±0.11 | µg/mL |
| Norvaline | 29.5±4.56 | ng/mL |
| Ornithine | 1.29±1.12 | µg/mL |
| Oxalic acid | 3.49±5.07 | µg/mL |
| Carbonyladipic acid | 0.72±0.41 | µg/mL |
| Oxoglutaric acid | 0.17±0.17 | µg/mL |
| Palmitic acid | 3.79±2.29 | µg/mL |
| Palmitoleic acid | 3.69±2.19 | µg/mL |
| Nonanoic acid | 87.4±12.5 | ng/mL |
| Pentadecanoic acid | 0.31±0.24 | µg/mL |
| Phenol | 58.7±6.78 | ng/mL |
| Phenylacetic acid | 1.49±1.14 | µg/mL |
| Phenyllactic acid | 71.5±8.79 | ng/mL |
| Pimelic acid | 0.31±0.22 | µg/mL |
| Propionic acid | 10.09±6.03 | µg/mL |
| Pyroglutamic acid | 2.6±3.95 | µg/mL |
| Stearic acid | 1.22±1.63 | µg/mL |
| Suberic acid | 0.11±0.03 | µg/mL |
| Succinic acid | 0.42±0.32 | µg/mL |
| Tartaric acid | 80.3±9.97 | ng/mL |
| Thiamine | 2.23±4.06 | µg/mL |
| Valeric acid | 1.39±1.36 | µg/mL |
| Vanillic acid | 0.17±0.04 | µg/mL |
| Cis-aconitic acid | 70.05±10.08 | ng/mL |
| P-cresol | 0.24±0.19 | µg/mL |
| P-hydroxyphenylacetic acid | 0.3±0.22 | µg/mL |

## Claims

1. A quantitative detection method of multiple metabolic components in a biological sample, comprising performing derivatization treatment on the biological sample and then detecting the derivatized biological sample by liquid chromatography-mass spectrometry, wherein during derivatization treatment, 3-nitrophenylhydrazine is used as a derivatization reagent, and 1-(3-dimethylaminopropyl)-3-ethylcarbodiimide is used as a derivatization reaction catalyst; the biological sample is selected from urine, blood, cerebrospinal fluid, tissue, cells, saliva and fecal samples of a mammal; the multiple metabolic components in the biological sample are selected from one or more of amino acid, phenol, phenyl or benzyl derivative, indole, organic acid, fatty acid, sugar, and bile acid and have different magnitudes in content; wherein the method comprises the following steps:
a) collecting a biological sample;
b) extracting the biological sample with a mixed solvent of methanol, chloroform, and water, performing centrifugation, and then taking a supernatant, namely a biological sample extract;
c) adding the same volume of a 3-nitrophenylhydrazine methanol solution and a 1-(3-dimethylaminopropyl)-3-ethylcarbodiimide pyridine solution into the biological sample extract obtained in b), and performing uniform vortex mixing and heating for derivatization, wherein the concentration of used 3-nitrophenylhydrazine is 100-320 mmol/L, the concentration of 1-(3-dimethylaminopropyl)-3-ethylcarbodiimide is 50-200 mmol/L, the reaction temperature is 20-60°C, and the reaction time is 10-120 minutes;
d) adding a carbon-13 labeled isotope internal standard solution obtained from the reaction of 3-nitrophenylhydrazine and 1-(3-dimethylaminopropyl)-3-ethylcarbodiimide into the derivatized biological sample extract obtained in c); and
e) adding a methanol-water mixed solution into the sample in d) for dilution, and determining amino acid, phenol, phenyl or benzyl derivative, indole, organic acid, fatty acid, sugar, and bile acid by liquid chromatography-mass spectrometry.

2. The detection method according to claim 1, wherein in step c), the concentration of used 3-nitrophenylhydrazine is 150-220 mmol/L, the concentration of 1-(3-dimethylaminopropyl)-3-ethylcarbodiimide is 80-120 mmol/L, the reaction temperature is 20-40°C, and the reaction time is 30-60 minutes.

3. The detection method according to claim 1, wherein when the biological sample is urine, blood, saliva or cerebrospinal fluid, a treatment method of the biological sample in step b) comprises: taking an appropriate amount of the biological sample, extracting the biological sample with a mixed solvent of cold methanol, chloroform and water at a volume ratio of 3:1:1, shaking the mixture for a few seconds, performing centrifugation on the sample at a rotation speed of 10000-20000 rpm at a low temperature for 5-15 minutes, and transferring a supernatant into an autosampler glass vial for subsequent derivatization treatment.

4. The detection method according to claim 1, wherein when the biological sample is a fecal sample, a treatment method of the biological sample in step b) comprises: freeze-drying the fecal sample; homogenizing an appropriate amount of the freeze-dried fecal sample with an appropriate amount of a mixed solvent of cold methanol, chloroform and water at a volume ratio of 3:1:1, performing centrifugation on the sample at a rotation speed of 10000-20000 rpm at a low temperature for 15-30 minutes, and transferring a supernatant into an autosampler glass vial for subsequent derivatization treatment.

5. The detection method according to claim 1, wherein when the biological sample is a tissue or cell sample, a treatment method of the biological sample in step b) comprises: adding an appropriate amount of a mixed solvent of cold methanol, chloroform and water at a volume ratio of 3:1:1 into the sample for homogenization, performing centrifugation on the sample at a rotation speed of 10000-20000 rpm at 4°C for 15-30 minutes, and transferring a supernatant into an autosampler glass vial for subsequent derivatization treatment.

6. The detection method according to claim 1, wherein the volume ratio of methanol to water in step e) is 1:1.

7. The detection method according to any one of claims 1 to 6, wherein the multiple metabolic components of the biological sample may be selected from the following multiple metabolites: fructose 1,6-diphosphate, 10Z-nonadecenoic acid, trans-11-octadecenoic acid, cis-11-octadecenoic acid, 12-dehydrocholic acid, 12-hydroxystearic acid, 12-ketolithocholic acid, 1-methylhistidine, 2,2-dimethylsuccinic acid, 2,3-diaminopropionic acid, glucoside 24-chenodeoxycholic acid, 2-butenoic acid, 2-oxoadipic acid, 2-methyl-4-pentenoic acid, 2-methyl-β-alanine, 2-methylbutyric acid, 2-methylhexanoic acid, 2-methylglutaric acid, 2-methylglutamic acid, 2-furoic acid, 2-hydroxy-2-methylbutyric acid, 2-hydroxy-3-methylbutyric acid, 2-hydroxybutyric acid, 2-hydroxycaproic acid, 2-hydroxycinnamic acid, 2-hydroxyphenylacetic acid, 2-hydroxyhippuric acid, 2-phenylpropionic acid, 2-phenylglycine, 3-(3-hydroxyphenyl)-3-hydroxypropionic acid, 3-(4-hydroxyphenyl)lactic acid, 3,4-dihydroxymandelic acid, 3,4-dihydroxyphenylpropionic acid, 3,4 -dehydro-DL-proline, 3,5-diiodo-L-thyroxine, 3β-cholic acid, 3-pyridineacetic acid, 3-indoleacrylic acid, 3-indolepropionic acid, 3-indoleacetamide, 3-oxyalanine, 3-aminosalicylic acid, 3-chloro-L-tyrosine, 3-methyl-2-oxobutyric acid, 3-methyl-2-oxovaleric acid, 3-methylindole, 3-methyladipic acid, 3-methylglutamic acid, 3-nitrotyrosine, sulfate 3-taurolithocholic acid, sulfate 3-lithocholic acid, 3-hydroxy-2-aminobenzoic acid, 3-hydroxybutyric acid, 3-hydroxypropionic acid, 3-hydroxyisovaleric acid, 3-hydroxyphenylacetic acid, 3-hydroxyhippuric acid, 3-dehydrocholic acid, 3-phenylbutyric acid, 4-methyl-2-oxovaleric acid, 4-methylhexanoic acid, 4-methoxyphenylacetic acid, 4-hydroxy-3-methoxymandelic acid, 4-hydroxycinnamic acid, 4-hydroxybenzoic acid, 4-hydroxyhippuric acid, 4-hydroxyphenyllactic acid, 4-hydroxyphenylpyruvic acid, 4-phenylbutyric acid, 5-aminolevulinic acid, 5-hydroxytryptophan, 5-hydroxytryptamine, 5-hydroxylysine, 6,7-diketolithocholic acid, 6-phosphogluconic acid, 6-ketolithocholic acid, 7,12-diketolithocholic acid, 7-ketolithocholic acid, 7-ketodeoxycholic acid, 9,11-conjugated linoleic acid, D-2-hydroxyglutaric acid, D-galactose, D-xylose, D-xylulose, D-fructose, D-ribose, D-ribose-5-phosphate, D-ribulose, D-ribulose-5-phosphate, D-mannose, D-glucose, D-maltose, L-2-aminobutyric acid, L-3-phenyllactic acid, L-alanine, L-serine, L-acetylcamitine, L-lactic acid, L-allothreonine, L-cysteine, L-homoserine, L-homocysteine, L-homocitrulline, L-pipecolic acid, L-aspartic acid, L-asparagine, L-sorbose, L-lignic acid, L-norleucine, L-kynurenine, L-thyronine, L-arginine, L-histidine, L-valine, L-cystine, L-cystathionine, L-proline, L-tryptophan, L-threonine, L-phenylalanine, L-malic acid, L-methionine, L-glutamine, L-glutamic acid, L-lysine, L-tyrosine, L-arabinose, N-(3-phenylpropionyl)glycine, N-acetyl-L-alanine, N-acetyl-L-aspartic acid, N-acetyl-L-phenylalanine, N-acetyl-L-methionine, N-acetyl-L-tyrosine, N-acetylserine, N-acetyl-D-glucosamine, N-acetyl-L-phenylalanine, N-acetylmannosamine, N-acetylneuraminic acid, N-acetylhistidine, N-acetylhydroxytryptamine, N-acetyltryptophan, N-acetylglutamine, N-acetyllysine, N-acetylornithine, N-methylnicotinamide, N-phenylacetyl-glutamine, N-phenylacetylphenylalanine, S-adenosine homocysteine, α-D-glucose, α-lactose, α-linolenic acid, α-hydroxyisobutyric acid, α-ketoglutaric acid, α-muricholic acid, β-D-trehalose, β-alanine, β-ursocholic acid, β-muricholic acid, γ-L-glutamyl-L-alanine, γ-linolenic acid, γ-aminobutyric acid, ω-muricholic acid, butyric acid, trimethylamine nitroxide, adenosine triphosphate, malonic acid, propionic acid, acetoacetic acid, acetylcysteine, acetylglycine, acetylornithine, acetic acid, guanidine acetate, glycolic acid, lactulose, lactoylglutathione, heneicosanoic acid, cis-12-heneicosenoic acid, heptacosanoic acid, tricosanoic acid, cis-14-tricosenoic acid, docosanoic acid, docosatrienoic acid, cis-13,16-docosadienoic acid, docosapentaenoic acid, docosahexaenoic acid, docosatetraenoic acid, trans-13-docosaenoic acid, cis-13-docosaenoic acid, pentacosanoic acid, octacosanoic acid, hexacosanoic acid, tetracosanoic acid, eicosanoic acid, eicosatrienoic acid, eicosadienoic acid, eicosapentaenoic acid, trans-11-eicosenoic acid, cis-11-eicosenoic acid, cis-5-eicosenoic acid, cis-8-eicosenoic acid, dimethylglycine, adenosine diphosphate, linoleic acid, leucine, alloisoleucine, allolithocholic acid, allocholic acid, undecenoic acid, heptadecanoic acid, tridecanoic acid, nonadecanoic acid, nonadecadienoic acid, pentadecanoic acid, galactonic acid, galactitol, mecysteine, protocatechuic acid, apocholic acid, dehydrolithocholic acid, nordeoxycholic acid, trans-9-tetradecenoic acid, trans-aconitic acid, trans-4-hydroxyproline, trans-9-heptadecenoic acid, trans-9-pentadecenoic acid, trans-9-hexadecenoic acid, trans-linolenic acid, trans-cinnamic acid, elaidic acid, homocysteine, pyrrole-2-carboxylic acid, picolinic acid, indole, indole-3-methyl acetate, indole-3-carboxylic acid, indoleacetic acid, purine, azelaic acid, nonanoic acid, dopa, dopamine, melibiose, fumaric acid, acetaminophen, p-aminohippuric acid, p-cresol sulfate, symmetrical dimethylarginine, p-hydroxymandelic acid, p-hydroxyphenylacetic acid, homogentisic acid, adipic acid, pimelic acid, isobutyric acid, isoleucine, isovaleric acid, citric acid, isoursodeoxycholic acid, isohyodeoxycholic acid, isolithocholic acid, isocholic acid, isodeoxycholic acid, glutaric acid, glutaconic acid, valeric acid, mandelic acid, lauric acid, fructose-6-phosphate, citraconic acid, citric acid, citramalic acid, ribonolactone, ribonic acid, raffinose, palmitoleic acid, palmitic acid, norvaline, n-hydroxyphenylacetic acid, oxidized glutathione, aminoadipic acid, aminocaproic acid, salicyluric acid, oleic acid, trehalose, nicotinic acid, pyroglutamic acid, ursocholic acid, ursodeoxycholic acid, tauro-α-muricholic acid, tauro-b-muricholic acid, tauro-w-muricholic acid, tauroursodeoxycholic acid, taurohyocholic acid, taurohyodeoxycholic acid, taurolithocholic acid, taurocholic acid, taurodehydrocholic acid, taurochenodeoxycholic acid, hyocholic acid, hyodeoxycholic acid, succinylacetone, succinic acid, citrulline, glycodehydrocholic acid, glycolithocholic acid, glycodeoxycholic acid, glycine, glycochenodeoxycholic acid, glyceraldehyde, glyceraldehyde-3-phosphate, choline glycerophosphate, glycoursodeoxycholic acid, glycohyocholic acid, glycohyodeoxycholic acid, glycocholic acid, glyproline, glycyl-L-leucine, mannose-6-phosphoric acid, mannitol, methylmalonic acid, methylsuccinic acid, formic acid, capric acid, lithocholic acid, selenomethionine, thiamine, glycolithocholic sulfate, stearic acid, liothyronine, dihydroxyacetone phosphate, phosphoribosyl pyrophosphate, creatine phosphate, hydroxypyruvic acid, glycine hydroxyphenylacetate, cinnamic acid, sarcosine, carnosine, creatine, inositol, epinephrine, choline, cholic acid, dehydrocholic acid, demethylcholic acid, adenosine monophosphate, arachidonic acid, phenylpyruvic acid, phenethylamine, phenylpropionic acid, phenyllactic acid, benzamide, benzoic acid, oxalic acid, shikimic acid, glucaric acid, glucose-6-phosphate, glucose lactone, sebacic acid, ricinoleic acid, sucrose, methionine sulfoxide, itaconic acid, melatonin, glutathione, myristic acid, erythronic acid, erythrose, suberic acid, caprylic acid, phthalic acid, tartaric acid, tyramine, quinic acid, m-aminobenzoic acid, asymmetric dimethylarginine, tannic acid, cis-10,12-octadecadienoic acid, cis-12,15-heneicosadienoic acid, cis-12-tridecenoic acid, cis-15-tetracosenic acid, cis-2-hydroxycinnamic acid, cis-9-tetradecenoic acid, cis-10-heptadecenoic acid, cis-11-dodecenoic acid, cis-4-hydroxyproline, cis-5-dodecenoic acid, cis-7-hexadecenoic acid, cis-9-heptadecenoic acid, cis-aconitic acid, vanillic acid, hippuric acid, maleic acid, homovanillic acid, ornithine, guanosine monophosphate, guanosine triphosphate, anserine, chenodeoxycholic acid, maltotriose, maltitol, rhamnose and murideoxycholic acid.

8. Use of a metabolic chip in the detection method according to any one of claims 1 to 7, the metabolic chip comprising a chip carrier, a filter device and dry solid powder of a standard product and a quality control product, wherein the chip carrier is a microtiter plate, each well of the microtiter plate is provided with an independent filter device, and the powder obtained by dehydrating or freeze-drying solutions of the standard product and the quality control product is placed on the filter device in each well of the microtiter plate; the standard product is selected from one or more of amino acid, phenol, phenyl or benzyl derivative, indole, organic acid, fatty acid, sugar, and bile acid standard products; the quality control product is selected from one or more of amino acid, phenol, phenyl or benzyl derivative, indole, organic acid, fatty acid, sugar, and bile acid standard products corresponding to the standard products above.

9. Use of the metabolic chip according to claim 8, wherein the microtiter plate is selected from a 48-well plate, a 96-well plate and a 384-well plate, and the filter device is a filter membrane made of polyvinylidene fluoride, cellulose acetate, or nylon with a pore size of 0.20-0.45 micron.

10. Use of the metabolic chip according to claim 8, wherein the standard product and the quality control product may be selected from the following multiple metabolites: fructose 1,6-diphosphate, 10Z-nonadecenoic acid, trans-11-octadecenoic acid, cis-11-octadecenoic acid, 12-dehydrocholic acid, 12-hydroxystearic acid, 12-ketolithocholic acid, 1-methylhistidine, 2,2-dimethylsuccinic acid, 2,3-diaminopropionic acid, glucoside 24-chenodeoxycholic acid, 2-butenoic acid, 2-oxoadipic acid, 2-methyl-4-pentenoic acid, 2-methyl-β-alanine, 2-methylbutyric acid, 2-methylhexanoic acid, 2-methylglutaric acid, 2-methylglutamic acid, 2-furoic acid, 2-hydroxy-2-methylbutyric acid, 2-hydroxy-3-methylbutyric acid, 2-hydroxybutyric acid, 2-hydroxycaproic acid, 2-hydroxycinnamic acid, 2-hydroxyphenylacetic acid, 2-hydroxyhippuric acid, 2-phenylpropionic acid, 2-phenylglycine, 3-(3-hydroxyphenyl)-3-hydroxypropionic acid, 3-(4-hydroxyphenyl)lactic acid, 3,4-dihydroxymandelic acid, 3,4-dihydroxyphenylpropionic acid, 3,4-dehydro-DL-proline, 3,5-diiodo-L-thyroxine, 3β-cholic acid, 3-pyridineacetic acid, 3-indoleacrylic acid, 3-indolepropionic acid, 3-indoleacetamide, 3-oxyalanine, 3-aminosalicylic acid, 3-chloro-L-tyrosine, 3-methyl-2-oxobutyric acid, 3-methyl-2-oxovaleric acid, 3-methylindole, 3-methyladipic acid, 3-methylglutamic acid, 3-nitrotyrosine, sulfate 3-taurolithocholic acid, sulfate 3-lithocholic acid, 3-hydroxy-2-aminobenzoic acid, 3-hydroxybutyric acid, 3-hydroxypropionic acid, 3-hydroxyisovaleric acid, 3-hydroxyphenylacetic acid, 3-hydroxyhippuric acid, 3-dehydrocholic acid, 3-phenylbutyric acid, 4-methyl-2-oxyvaleric acid, 4-methylhexanoic acid, 4-methoxyphenylacetic acid, 4-hydroxy-3-methoxymandelic acid, 4-hydroxycinnamic acid, 4-hydroxybenzoic acid, 4-hydroxyhippuric acid, 4-hydroxyphenyllactic acid, 4-hydroxyphenylpyruvic acid, 4-phenylbutyric acid, 5-aminolevulinic acid, 5-hydroxytryptophan, 5-hydroxytryptamine, 5-hydroxylysine, 6,7-diketolithocholic acid, 6-phosphogluconic acid, 6-ketolithocholic acid, 7,12-diketolithocholic acid, 7-ketolithocholic acid, 7-ketodeoxycholic acid, 9,11-conjugated linoleic acid, D-2-hydroxyglutaric acid, D-galactose, D-xylose, D-xylulose, D-fructose, D-ribose, D-ribose-5-phosphate, D-ribulose, D-ribulose-5-phosphate, D-mannose, D-glucose, D-maltose, L-2-aminobutyric acid, L-3-phenyllactic acid, L-alanine, L-serine, L-acetylcarnitine, L-lactic acid, L-allothreonine, L-cysteine, L-homoserine, L-homocysteine, L-homocitrulline, L-pipecolic acid, L-aspartic acid, L-asparagine, L-sorbose, L-lignic acid, L-norleucine, L-kynurenine, L-thyronine, L-arginine, L-histidine, L-valine, L-cystine, L-cystathionine, L-proline, L-tryptophan, L-threonine, L-phenylalanine, L-malic acid, L-methionine, L-glutamine, L-glutamic acid, L-lysine, L-tyrosine, L-arabinose, N-(3-phenylpropionyl)glycine, N-acetyl-L-alanine, N-acetyl-L-aspartic acid, N-acetyl-L-phenylalanine, N-acetyl-L-methionine, N-acetyl-L-tyrosine, N-acetylserine, N-acetyl-D-glucosamine, N-acetyl-L-phenylalanine, N-acetylmannosamine, N-acetylneuraminic acid, N-acetylhistidine, N-acetylhydroxytryptamine, N-acetyltryptophan, N-acetylglutamine, N-acetyllysine, N-acetylornithine, N-methylnicotinamide, N-phenylacetyl-glutamine, N-phenylacetylphenylalanine, S-adenosine homocysteine, α-D-glucose, α-lactose, α-linolenic acid, α-hydroxyisobutyric acid, α-ketoglutaric acid, α-muricholic acid, β-D-trehalose, β-alanine, β-ursocholic acid, β-muricholic acid, γ-L-glutamyl-L-alanine, γ-linolenic acid, γ-aminobutyric acid, ω-muricholic acid, butyric acid, trimethylamine nitroxides, adenosine triphosphate, malonic acid, propionic acid, acetoacetic acid, acetylcysteine, acetylglycine, acetylornithine, acetic acid, guanidine acetate, glycolic acid, lactulose, lactoylglutathione, heneicosanoic acid, cis-12-heneicosenoic acid, heptacosanoic acid, tricosanoic acid, cis-14-tricosenoic acid, docosanoic acid, docosatrienoic acid, cis-13,16-docosadienoic acid, docosapentaenoic acid, docosahexaenoic acid, docosatetraenoic acid, trans-13-docosaenoic acid, cis-13-docosaenoic acid, pentacosanoic acid, octacosanoic acid, hexacosanoic acid, tetracosanoic acid, eicosanoic acid, eicosatrienoic acid, eicosadienoic acid, eicosapentaenoic acid, trans-11-eicosenoic acid, cis-11-eicosenoic acid, cis-5-eicosenoic acid, cis-8-eicosenoic acid, dimethylglycine, adenosine diphosphate, linoleic acid, leucine, alloisoleucine, allolithocholic acid, allocholic acid, undecenoic acid, heptadecanoic acid, tridecanoic acid, nonadecanoic acid, nonadecadienoic acid, pentadecanoic acid, galactonic acid, galactitol, mecysteine, protocatechuic acid, apocholic acid, dehydrolithocholic acid, nordeoxycholic acid, trans-9-tetradecenoic acid, trans-aconitic acid, trans-4-hydroxyproline, trans-9-heptadecenoic acid, trans-9-pentadecenoic acid, trans-9-hexadecenoic acid, trans-linolenic acid, trans-cinnamic acid, elaidic acid, homocysteine, pyrrole-2-carboxylic acid, picolinic acid, indole, indole-3-methyl acetate, indole-3-carboxylic acid, indoleacetic acid, purine, azelaic acid, nonanoic acid, dopa, dopamine, melibiose, fumaric acid, acetaminophen, p-aminohippuric acid, p-cresol sulfate, symmetrical dimethylarginine, p-hydroxymandelic acid, p-hydroxyphenylacetic acid, homogentisic acid, adipic acid, pimelic acid, isobutyric acid, isoleucine, isovaleric acid, citric acid, isoursodeoxycholic acid, isohyodeoxycholic acid, isolithocholic acid, isocholic acid, isodeoxycholic acid, glutaric acid, glutaconic acid, valeric acid, mandelic acid, lauric acid, fructose-6-phosphate, citraconic acid, citric acid, citramalic acid, ribonolactone, ribonic acid, raffinose, palmitoleic acid, palmitic acid, norvaline, n-hydroxyphenylacetic acid, oxidized glutathione, aminoadipic acid, aminocaproic acid, salicyluric acid, oleic acid, trehalose, nicotinic acid, pyroglutamic acid, ursocholic acid, ursodeoxycholic acid, tauro-α-muricholic acid, tauro-b-muricholic acid, tauro-w-muricholic acid, tauroursodeoxycholic acid, taurohyocholic acid, taurohyodeoxycholic acid, taurolithocholic acid, taurocholic acid, taurodehydrocholic acid, taurochenodeoxycholic acid, hyocholic acid, hyodeoxycholic acid, succinylacetone, succinic acid, citrulline, glycodehydrocholic acid, glycolithocholic acid, glycodeoxycholic acid, glycine, glycochenodeoxycholic acid, glyceraldehyde, glyceraldehyde-3-phosphate, choline glycerophosphate, glycoursodeoxycholic acid, glycohyocholic acid, glycohyodeoxycholic acid, glycocholic acid, glyproline, glycyl-L-leucine, mannose-6-phosphoric acid, mannitol, methylmalonic acid, methylsuccinic acid, formic acid, capric acid, lithocholic acid, selenomethionine, thiamine, glycolithocholic sulfate, stearic acid, liothyronine, dihydroxyacetone phosphate, phosphoribosyl pyrophosphate, creatine phosphate, hydroxypyruvic acid, glycine hydroxyphenylacetate, cinnamic acid, sarcosine, carnosine, creatine, inositol, epinephrine, choline, cholic acid, dehydrocholic acid, demethylcholic acid, adenosine monophosphate, arachidonic acid, phenylpyruvic acid, phenethylamine, phenylpropionic acid, phenyllactic acid, benzamide, benzoic acid, oxalic acid, shikimic acid, glucaric acid, glucose-6-phosphate, glucose lactone, sebacic acid, ricinoleic acid, sucrose, methionine sulfoxide, itaconic acid, melatonin, glutathione, myristic acid, erythronic acid, erythrose, suberic acid, caprylic acid, phthalic acid, tartaric acid, tyramine, quinic acid, m-aminobenzoic acid, asymmetric dimethylarginine, tannic acid, cis-10,12-octadecadienoic acid, cis-12,15-heneicosadienoic acid, cis-12-tridecenoic acid, cis-15-tetracosenic acid, cis-2-hydroxycinnamic acid, cis-9-tetradecenoic acid, cis-10-heptadecenoic acid, cis-11-dodecenoic acid, cis-4-hydroxyproline, cis-5-dodecenoic acid, cis-7-hexadecenoic acid, cis-9-heptadecenoic acid, cis-aconitic acid, vanillic acid, hippuric acid, maleic acid, homovanillic acid, ornithine, guanosine monophosphate, guanosine triphosphate, anserine, chenodeoxycholic acid, maltotriose, maltitol, rhamnose and murideoxycholic acid.

11. The use of the metabolic chip according to any one of claims 8 to 10, comprising the following steps:
a) collecting a biological sample;
b) according to the sample type, preparing a corresponding biological sample extract by using the corresponding method according to claim 3 or 4 or 5;
c) adding the prepared biological sample extract into each well of the metabolic chip in an equal amount, adding the same volume of a 3-nitrophenylhydrazine methanol solution and a 1-(3-dimethylaminopropyl)-3-ethylcarbodiimide pyridine solution into each well, and performing uniform vortex mixing and heating for derivatization, wherein the concentration of used 3-nitrophenylhydrazine is 100-320 mmol/L, the concentration of 1-(3-dimethylaminopropyl)-3-ethylcarbodiimide is 50-200 mmol/L, the reaction temperature is 20-60°C, and the reaction time is 10-120 minutes;
d) adding a carbon-13 labeled isotope internal standard solution obtained from the reaction of 3-nitrophenylhydrazine and 1-(3-dimethylaminopropyl)-3-ethylcarbodiimide into the derivatized biological sample extract obtained in c); and
e) adding a methanol-water mixed solution into each well in the metabolic chip in d) for dilution, placing the metabolic chip in a tandem mass spectrometer for determination of amino acid, phenol, phenyl or benzyl derivative, indole, organic acid, fatty acid, sugar, and bile acid by liquid chromatography-mass spectrometry, and calculating concentrations of target metabolites in the sample based on results.

## Patentansprüche

1. Verfahren zum quantitativen Nachweis von mehreren Stoffwechselkomponenten in einer biologischen Probe, umfassend das Durchführen einer Derivatisierungsbehandlung an der biologischen Probe und dann das Nachweisen der derivatisierten biologischen Probe mittels Flüssigchromatographie-Massenspektrometrie, wobei während der Derivatisierungsbehandlung 3-Nitrophenylhydrazin als Derivatisierungsreagenz und 1-(3-Dimethylaminopropyl)-3-Ethylcarbodiimid als Derivatisierungsreaktionskatalysator verwendet wird; die biologische Probe aus Urin-, Blut-, Zerebrospinalflüssigkeit-, Gewebe-, Zell-, Speichel- und Stuhlproben eines Säugetiers ausgewählt wird; wobei die mehreren Stoffwechselkomponenten in der biologischen Probe aus einem oder mehreren von Aminosäure-, Phenol-, Phenyl- oder Benzylderivat, Indol, organischen Säure, Fettsäure, Zucker und Gallensäure ausgewählt sind und im Gehalt unterschiedliche Größenordnungen aufweisen; wobei das Verfahren die folgenden Schritte umfasst:
a) Sammeln einer biologischen Probe;
b) Extrahieren der biologischen Probe mit einem gemischten Lösungsmittel bestehend aus Methanol, Chloroform und Wasser, Durchführen einer Zentrifugation und dann Entnehmen eines Überstandes, nämlich eines Extrakts der biologischen Probe;
c) Zugeben des gleichen Volumens einer 3-Nitrophenylhydrazin-Methanol-Lösung und einer 1-(3-Dimethylaminopropyl)-3-Ethylcarbodiimid-Pyridin-Lösung zu dem in Schritt b) erhaltenen Extrakt der biologischen Probe, und Durchführen eines gleichmäßigen Wirbelmischens und Erwärmens zur Derivatisierung, wobei die Konzentration an verwendetem 3-Nitrophenylhydrazin 100-320 mmol/L, die Konzentration an 1-(3-Dimethylaminopropyl)-3-Ethylcarbodiimid 50-200 mmol/L, die Reaktionstemperatur 20-60°C, und die Reaktionszeit 10-120 Minuten beträgt;
d) Zugeben einer mit Kohlenstoff-13-markierten Isotopen-Interne-Standard-Lösung, die aus der Reaktion von 3-Nitrophenylhydrazin und 1-(3-Dimethylaminopropyl)-3-Ethylcarbodiimid erhalten wurde, zu dem in Schritt c) erhaltenen derivatisierten Extrakt der biologischen Probe; und
e) Zugeben einer Methanol-Wasser-Mischlösung zu der Probe in Schritt d) zur Verdünnung und Bestimmen von Aminosäure, Phenol-, Phenyl- oder Benzylderivat, Indol, organischer Säure, Fettsäure, Zucker und Gallensäure durch Flüssigchromatographie-Massenspektrometrie.

2. Verfahren zum Nachweis nach Anspruch 1, wobei in Schritt c) die Konzentration an verwendetem 3-Nitrophenylhydrazin 150-220 mmol/L, die Konzentration an 1-(3-Dimethylaminopropyl)-3-Ethylcarbodiimid 80-120 mmol/L, die Reaktionstemperatur 20-40°C, und die Reaktionszeit 30-60 Minuten beträgt.

3. Verfahren zum Nachweis nach Anspruch 1, wobei bei der biologischen Probe Urin, Blut, Speichel oder Zerebrospinalflüssigkeit ist, ein Behandlungsverfahren der biologischen Probe in Schritt b) umfasst: Entnehmen einer geeigneten Menge der biologischen Probe, Extrahieren der biologischen Probe mit einem gemischten Lösungsmittel bestehend aus kaltem Methanol, Chloroform und Wasser bei einem Volumenverhältnis von 3:1:1, Schütteln der Mischung für einige Sekunden, Durchführen einer Zentrifugation an der Probe bei einer Rotationsgeschwindigkeit von 10000-20000 U/min bei einer niedrigen Temperatur für 5-15 Minuten, und Überführen eines Überstandes in ein Autosampler-Glasgefäß zur anschließenden Derivatisierungsbehandlung.

4. Verfahren zum Nachweis nach Anspruch 1, wobei bei der biologischen Probe eine Stuhlprobe ist, ein Behandlungsverfahren der biologischen Probe in Schritt b) umfasst: Gefriertrocknen der Stuhlprobe; Homogenisieren einer geeigneten Menge der gefriergetrockneten Stuhlprobe mit einer geeigneten Menge eines gemischten Lösungsmittels bestehend aus kaltem Methanol, Chloroform und Wasser in einem Volumenverhältnis von 3:1:1, Durchführen einer Zentrifugation an der Probe bei einer Rotationsgeschwindigkeit von 10000-20000 U/min bei einer niedrigen Temperatur für 15-30 Minuten und Überführen eines Überstandes in ein Autosampler-Glasgefäß zur anschließenden Derivatisierungsbehandlung.

5. Verfahren zum Nachweis nach Anspruch 1, wobei bei der biologischen Probe eine Gewebe- oder Zellprobe ist, ein Behandlungsverfahren der biologischen Probe in Schritt b) umfasst: Zugeben einer geeigneten Menge eines gemischten Lösungsmittels bestehend aus kaltem Methanol, Chloroform und Wasser bei einem Volumenverhältnis von 3:1:1 zur Probe zur Homogenisierung, Durchführen einer Zentrifugation an der Probe bei einer Rotationsgeschwindigkeit von 10000-20000 U/min bei 4°C für 15-30 Minuten, und Überführen eines Überstandes in ein Autosampler-Glasgefäß zur anschließenden Derivatisierungsbehandlung.

6. Verfahren zum Nachweis nach Anspruch 1, wobei das Volumenverhältnis von Methanol zu Wasser in Schritt e) 1:1 beträgt.

7. Verfahren zum Nachweis nach einem der Ansprüche 1 bis 6, wobei die mehreren Stoffwechselkomponenten der biologischen Probe unter den folgenden mehreren Metaboliten ausgewählt werden können: Fructose-1,6-Diphosphat, 10Z-Nonadecensäure, trans-11-Octadecensäure, cis-11-Octadecensäure, 12-Dehydrocholsäure, 12-Hydroxystearinsäure, 12-Ketolithocholsäure, 1-Methylhistidin, 2,2-Dimethylbernsteinsäure, 2,3-Diaminopropionsäure, Glucosid 24-Chenodesoxycholsäure, 2-Butensäure, 2-Oxoadipinsäure, 2-Methyl-4-pentensäure, 2-Methyl-β-Alanin, 2-Methylbuttersäure, 2-Methylhexansäure, 2-Methylglutarsäure, 2-Methylglutaminsäure, 2-Furonsäure, 2-Hydroxy-2-Methylbuttersäure, 2-Hydroxy-3-Methylbuttersäure, 2-Hydroxybuttersäure, 2-Hydroxycapronsäure, 2-Hydroxyzimtsäure, 2-Hydroxyphenylessigsäure, 2-Hydroxyhippursäure, 2-Phenylpropionsäure, 2-Phenylglycin, 3-(3-Hydroxyphenyl)-3-hydroxypropionsäure, 3-(4-Hydroxyphenyl)Milchsäure, 3,4-Dihydroxymandelsäure, 3,4-Dihydroxyphenylpropionsäure, 3,4-Dehydro-DL-Prolin, 3,5-Diiodo-L-Thyroxin, 3β-Cholsäure, 3-Pyridinessigsäure, 3-Indolacrylsäure, 3-Indolpropionsäure, 3-Indolacetamid, 3-Oxyalanin, 3-Aminosalicylsäure, 3-Chlor-L-Tyrosin, 3-Methyl-2-Oxobuttersäure, 3-Methyl-2-Oxovaleriansäure, 3-Methylindol, 3-Methyladipinsäure, 3-Methylglutaminsäure, 3-Nitrotyrosin, Sulfat-3-Taurocholsäure, Sulfat-3-Lithocholsäure, 3-Hydroxy-2-Aminobenzoesäure, 3-Hydroxybuttersäure, 3-Hydroxypropionsäure, 3-Hydroxyisovaleriansäure, 3-Hydroxyphenylessigsäure, 3-Hydroxyhippursäure, 3-Dehydrocholsäure, 3-Phenylbuttersäure, 4-Methyl-2-Oxovaleriansäure, 4-Methylhexansäure, 4-Methoxyphenylessigsäure, 4-Hydroxy-3-Methoxymandelsäure, 4-Hydroxyzimtsäure, 4-Hydroxybenzoesäure, 4-Hydroxyhippursäure, 4-Hydroxyphenylmilchsäure, 4-Hydroxyphenylbuttersäure, 4-Phenylbuttersäure, 5-Aminolävulinsäure, 5-Hydroxytryptophan, 5-Hydroxytryptamin, 5-Hydroxylysin, 6,7-Diketholithocholsäure, 6-Phosphogluconsäure, 6-Ketolithocholsäure, 7,12-Diketholithocholsäure, 7-Ketolithocholsäure, 7-Ketodeoxycholsäure, 9,11-konjugierte Linolsäure, D-2-Hydroxyglutarsäure, D-Galaktose, D-Xylose, D-Xylulose, D-Fruktose, D-Ribose, D-Ribose-5-Phosphat, D-Ribulose, D-Ribulose-5-Phosphat, D-Mannose, D-Glukose, D-Maltose, L-2-Aminobuttersäure, L-3-Phenylmilchsäure, L-Alanin, L-Serin, L-Acetylcamitin, L-Milchsäure, L-Allothreonin, L-Cystein, L-Homoserin, L-Homocystein, L-Homocitrullin, L-Pipecolsäure, L-Asparaginsäure, L-Asparagin, L-Sorbose, L-Ligninsäure, L-Norleucin, L-Kynurenin, L-Thyronin, L-Arginin, L-Histidin, L-Valin, L-Cystin, L-Cystathionin, L-Prolin, L-Tryptophan, L-Threonin, L-Phenylalanin, L-Maleinsäure, L-Methionin, L-Glutamin, L-Glutaminsäure, L-Lysin, L-Tyrosin, L-Arabinose, N-(3-Phenylpropionyl)Glycin, N-Acetyl-L-Alanin, N-Acetyl-L-Asparaginsäure, N-Acetyl-L-Phenylalanin, N-Acetyl-L-Methionin, N-Acetyl-L-Tyrosin, N-Acetylserin, N-Acetyl-D-Glucosamin, N-Acetyl-L-Phenylalanin, N-Acetylmannosamin, N-Acetylneuraminsäure, N-Acetylhistidin, N-Acetylhydroxytryptamin, N-Acetyltryptophan, N-Acetylglutamin, N-Acetyllysin, N-Acetylornithin, N-Methylnicotinamid, N-Phenylacetylglutamin, N-Phenylacetylphenylalanin, S-Adenosinhomocystein, α-D-Glucose, α-Lactose, α-Linolensäure, α-Hydroxyisobuttersäure, α-Ketoglutarsäure, α-Muricholsäure, β-D-Trehalose, β-Alanin, β-Ursocolsäure, β-Muricholsäure, γ-L-Glutamyl-L-Alanin, γ-Linolensäure, γ-Aminobuttersäure, ω-Muricholsäure, Buttersäure, Trimethylamin-Nitroxid, Adenosintriphosphat, Malonsäure, Propionsäure, Acetoessigsäure, Acetylcystein, Acetylglycin, Acetylornithin, Essigsäure, Guanidinacetat, Glykolsäure, Lactulose, Lactoylglutathion, Heneicosansäure, cis-12-Heneicosensäure, Heptacosansäure, Tricosansäure, cis-14-Tricosensäure, Docosansäure, Docosatriensäure, cis-13,16-Docosadiensäure, Docosapentaensäure, Docosahexaensäure, Docosatetraensäure, trans-13-Docosaensäure, cis-13-Docosaensäure, Pentacosanolsäure, Octacosanolsäure, Hexacosanolsäure, Tetracosanolsäure, Eicosansäure, Eicosatriensäure, Eicosadiensäure, Eicosapentaensäure, trans-11-Eicosensäure, cis-11-Eicosensäure, cis-5-Eicosensäure, cis-8-Eicosensäure, Dimethylglycin, Adenosindiphosphat, Linolsäure, Leucin, Alloisoleucin, Allolithocholsäure, Allocholsäure, Undecensäure, Heptadecansäure, Tridecansäure, Nonadecansäure, Nonadecadiensäure, Pentadecansäure, Galaktonsäure, Galaktit, Mecystein, Protocatechusäure, Apocholsäure, Dehydrolithocholsäure, Nordeoxycholsäure, trans-9-Tetradecensäure, trans-Aconitsäure, trans-4-Hydroxyprolin, trans-9-Heptadecensäure, trans-9-Pentadecensäure, trans-9-Hexadecensäure, trans-Linolensäure, trans-Zimtsäure, Elaidinsäure, Homocystein, Pyrrol-2-Carbonsäure, Picolinsäure, Indol, Indol-3-Methylacetat, Indol-3-Carbonsäure, Indolessigsäure, Purin, Azelainsäure, Nonansäure, Dopa, Dopamin, Melibiose, Fumarsäure, Acetaminophen, p-Aminohippursäure, p-Kresolsulfat, symmetrisches Dimethylarginin, p-Hydroxymandelsäure, p-Hydroxyphenylessigsäure, Homogentisinsäure, Adipinsäure, Pimelinsäure, Isobuttersäure, Isoleucin, Isovaleriansäure, Zitronensäure, Isoursodeoxycholsäure, Isohyodeoxycholsäure, Isolithocholsäure, Isocholsäure, Isodeoxycholsäure, Glutarsäure, Glutaconsäure, Valeriansäure, Mandelsäure, Laurinsäure, Fructose-6-Phosphat, Citraconinsäure, Zitronensäure, Citramalsäure, Ribonolacton, Ribonsäure, Raffinose, Palmitoleinsäure, Palmitinsäure, Norvalin, n-Hydroxyphenylessigsäure, Oxidiertes Glutathion, Aminoadipinsäure, Aminocapronsäure, Salicylsäure, Oleinsäure, Trehalose, Nikotinsäure, Pyroglutaminsäure, Ursocholsäure, Ursodeoxycholsäure, Tauro-a-Muricholsäure, Tauro-b-Muricholsäure, Tauro-w-Muricholsäure, Tauroursodeoxycholsäure, Taurohyocholsäure, Taurohyodeoxycholsäure, Taurolithocholsäure, Taurocholsäure, Taurodehydrocholsäure, Taurochenodeoxycholsäure, Hyocholsäure, Hyodeoxycholsäure, Succinylaceton, Bernsteinsäure, Citrullin, Glycodehydrocholsäure, Glykolithocholsäure, Glycodeoxycholsäure, Glycin, Glycochenodeoxycholsäure, Glyceraldehyd, Glyceraldehyd-3-phosphat, Cholin-Glycerophosphat, Glycoursodeoxycholsäure, Glycohyocholsäure, Glycohyodeoxycholsäure, Glycholsäure, Glyprolin, Glycyl-L-Leucin, Mannose-6-Phosphorsäure, Mannitol, Methylmalonsäure, Methylbernsteinsäure, Ameisensäure, Caprinsäure, Lithocholsäure, Selenomethionin, Thiamin, Glykolithocholsulfat, Stearinsäure, Liothyronin, Dihydroxyacetonphosphat, Phosphoribosylpyrophosphat, Kreatinphosphat, Hydroxypyruvinsäure, Glycinhydroxyphenylacetat, Zimtsäure, Sarcosin, Carnosin, Kreatin, Inositol, Epinephrin, Cholin, Cholsäure, Dehydrocholsäure, Demethylcholsäure, Adenosinmonophosphat, Arachidonsäure, Phenylbrenztraubelsäure, Phenethylamin, Phenylpropionsäure, Phenylmilchsäure, Benzamid, Benzoesäure, Oxalsäure, Shikimisäure, Glucarsäure, Glukose-6-Phosphat, Glucoselacton, Sebacinsäure, Ricinolsäure, Saccharose, Methioninsulfoxid, Itaconsäure, Melatonin, Glutathion, Myristinsäure, Erythronsäure, Erythrose, Suberinsäure, Caprylsäure, Phthalsäure, Weinsäure, Tyramin, Chinasäure, m-Aminobenzoesäure, Asymmetrisches Dimethylarginin, Gerbsäure, cis-10,12-Octadecadiensäure, cis-12,15-Heneicosadiensäure, cis-12-Tridecensäure, cis-15-Tetracosensäure, cis-2-Hydroxyzimtsäure, cis-9-Tetradecensäure, cis-10-Heptadecensäure, cis-11-Dodecensäure, cis-4-Hydroxyprolin, cis-5-Dodecensäure, cis-7-Hexadecensäure, cis-9-Heptadecensäure, cis-Aconitsäure, Vanillinsäure, Hippursäure, Maleinsäure, Homovanillinsäure, Ornithin, Guanosinmonophosphat, Guanosintriphosphat, Anserin, Chenodeoxycholsäure, Maltotriose, Maltitol, Rhamnose und Murideoxycholsäure.

8. Verwendung eines Stoffwechselchips in dem Verfahren zum Nachweis nach einem der Ansprüche 1 bis 7, wobei der Stoffwechselchip einen Chipträger, eine Filtervorrichtung und trockenes festes Pulver eines Standardprodukts und ein Qualitätskontrollprodukt umfasst, wobei der Chipträger eine Mikrotiterplatte ist, jedem Well der Mikrotiterplatte mit einer unabhängigen Filtervorrichtung versehen ist und das Pulver, das durch Dehydratisieren oder Gefriertrocknen von Lösungen des Standardprodukts und des Qualitätskontrollprodukts erhalten wird, in jedem Well der Mikrotiterplatte auf der Filtervorrichtung platziert wird; das Standardprodukt aus einem oder mehreren von Aminosäure-, Phenol-, Phenyl- oder Benzylderivat-, Indol-, organischen Säure-, Fettsäure-, Zucker- und Gallensäure-Standardprodukten ausgewählt ist; das Qualitätskontrollprodukt aus einem oder mehreren von Aminosäure-, Phenol-, Phenyl- oder Benzylderivat-, Indol-, organischen Säure-, Fettsäure-, Zucker- und Gallensäure-Standardprodukten ausgewählt ist, die den vorstehenden Standardprodukten entsprechen.

9. Verwendung des Stoffwechselchips nach Anspruch 8, wobei die Mikrotiterplatte aus einer 48-Well-Platte, einer 96-Well-Platte und einer 384-Well-Platte ausgewählt ist und die Filtervorrichtung eine Filtermembran aus Polyvinylidenfluorid, Celluloseacetat oder Nylon mit einer Porengröße von 0,20-0,45 Mikron ist.

10. Verwendung des Stoffwechselchips nach Anspruch 8, wobei das Standardprodukt und das Qualitätskontrollprodukt unter den folgenden mehreren Metaboliten ausgewählt werden können: Fructose-1,6-Diphosphat, 10Z-Nonadecensäure, trans-11-Octadecensäure, cis-11-Octadecensäure, 12-Dehydrocholsäure, 12-Hydroxystearinsäure, 12-Ketolithocholsäure, 1-Methylhistidin, 2,2-Dimethylbernsteinsäure, 2,3-Diaminopropionsäure, Glucosid 24-Chenodesoxycholsäure, 2-Butensäure, 2-Oxoadipinsäure, 2-Methyl-4-Pentensäure, 2-Methyl-β-Alanin, 2-Methylbuttersäure, 2-Methylhexansäure, 2-Methylglutarsäure, 2-Methylglutaminsäure, 2-Furonsäure, 2-Hydroxy-2-Methylbuttersäure, 2-Hydroxy-3-Methylbuttersäure, 2-Hydroxybuttersäure, 2-Hydroxycapronsäure, 2-Hydroxyzimtsäure, 2-Hydroxyphenylessigsäure, 2-Hydroxyhippursäure, 2-Phenylpropionsäure, 2-Phenylglycin, 3-(3-Hydroxyphenyl)-3-Hydroxypropionsäure, 3-(4-Hydroxyphenyl)Milchsäure, 3,4-Dihydroxymandelsäure, 3,4-Dihydroxyphenylpropionsäure, 3,4-Dehydro-DL-Prolin, 3,5-Diiodo-L-thyroxin, 3β-Cholsäure, 3-Pyridinessigsäure, 3-Indolacrylsäure, 3-Indolpropionsäure, 3-Indolacetamid, 3-Oxyalanin, 3-Aminosalicylsäure, 3-Chlor-L-tyrosin, 3-Methyl-2-Oxobuttersäure, 3-Methyl-2-Oxovaleriansäure, 3-Methylindol, 3-Methyladipinsäure, 3-Methylglutaminsäure, 3-Nitrotyrosin, Sulfat 3-Taurocholsäure, Sulfat 3-Lithocholsäure, 3-Hydroxy-2-Aminobenzoesäure, 3-Hydroxybuttersäure, 3-Hydroxypropionsäure, 3-Hydroxyisovaleriansäure, 3-Hydroxyphenylessigsäure, 3-Hydroxyhippursäure, 3-Dehydrocholsäure, 3-Phenylbuttersäure, 4-Methyl-2-Oxyvaleriansäure, 4-Methylhexansäure, 4-Methoxyphenylessigsäure, 4-Hydroxy-3-Methoxymandelsäure, 4-Hydroxyzimtsäure, 4-Hydroxybenzoesäure, 4-Hydroxyhippursäure, 4-Hydroxyphenylmilchsäure, 4-Hydroxyphenylbuttersäure, 4-Phenylbuttersäure, 5-Aminolävulinsäure, 5-Hydroxytryptophan, 5-Hydroxytryptamin, 5-Hydroxylysin, 6,7-Diketolithocholsäure, 6-Phosphogluconsäure, 6-Ketolithocholsäure, 7,12-Diketolithocholsäure, 7-Ketolithocholsäure, 7-Ketodeoxycholsäure, 9,11-konjugierte Linolsäure, D-2-Hydroxyglutarsäure, D-Galaktose, D-Xylose, D-Xylulose, D-Fructose, D-Ribose, D-Ribose-5-Phosphat, D-Ribulose, D-Ribulose-5-Phosphat, D-Mannose, D-Glucose, D-Maltose, L-2-Aminobuttersäure, L-3-Phenylmilchsäure, L-Alanin, L-Serin, L-Acetylcamitin, L-Milchsäure, L-Allothreonin, L-Cystein, L-Homoserin, L-Homocystein, L-Homocitrullin, L-Pipecolsäure, L-Asparaginsäure, L-Asparagin, L-Sorbose, L-Ligninsäure, L-Norleucin, L-Kynurenin, L-Thyronin, L-Arginin, L-Histidin, L-Valin, L-Cystin, L-Cystathionin, L-Prolin, L-Tryptophan, L-Threonin, L-Phenylalanin, L-Maleinsäure, L-Methionin, L-Glutamin, L-Glutaminsäure, L-Lysin, L-Tyrosin, L-Arabinose, N-(3-Phenylpropionyl)Glycin, N-Acetyl-L-Alanin, N-Acetyl-L-Asparaginsäure, N-Acetyl-L-Phenylalanin, N-Acetyl-L-Methionin, N-Acetyl-L-Tyrosin, N-Acetylserin, N-Acetyl-D-Glucosamin, N-Acetyl-L-Phenylalanin, N-Acetylmannosamin, N-Acetylneuraminsäure, N-Acetylhistidin, N-Acetylhydroxytryptamin, N-Acetyltryptophan, N-Acetylglutamin, N-Acetyllysin, N-Acetylornithin, N-Methylnicotinamid, N-Phenylacetylglutamin, N-Phenylacetylphenylalanin, S-Adenosinhomocystein, α-D-Glucose, α-Lactose, α-Linolensäure, α-Hydroxyisobuttersäure, α-Ketoglutarsäure, α-Muricholsäure, β-D-Trehalose, β-Alanin, β-Ursocolsäure, β-Muricholsäure, γ-L-Glutamyl-L-Alanin, γ-Linolensäure, γ-Aminobuttersäure, ω-Muricholsäure, Buttersäure, Trimethylamin-Nitroxide, Adenosintriphosphat, Malonsäure, Propionsäure, Acetoessigsäure, Acetylcystein, Acetylglycin, Acetylornithin, Essigsäure, Guanidinacetat, Glykolsäure, Lactulose, Lactoylglutathion, Heneicosansäure, cis-12-Heneicosensäure, Heptacosansäure, Tricosansäure, cis-14-Tricosensäure, Docosansäure, Docosatriensäure, cis-13,16-Docosadiensäure, Docosapentaensäure, Docosahexaensäure, Docosatetraensäure, trans-13-Docosensäure, cis-13-Docosensäure, Pentacosansäure, Octacosansäure, Hexacosansäure, Tetracosansäure, Eicosansäure, Eicosatriensäure, Eicosadiensäure, Eicosapentaensäure, trans-11-Eicosensäure, cis-11-Eicosensäure, cis-5-Eicosensäure, cis-8-Eicosensäure, Dimethylglycin, Adenosindiphosphat, Linolsäure, Leucin, Alloisoleucin, Allolithocholsäure, Allocholsäure, Undecensäure, Heptadecansäure, Tridecansäure, Nonadecansäure, Nonadecadiensäure, Pentadecansäure, Galactonsäure, Galactitol, Mecystein, Protocatechusäure, Apocholsäure, Dehydrolithocholsäure, Nordeoxycholsäure, trans-9-Tetradecensäure, trans-Aconitsäure, trans-4-Hydroxyprolin, trans-9-Heptadecensäure, trans-9-Pentadecensäure, trans-9-Hexadecensäure, trans-Linolensäure, trans-Zimtsäure, Elaidinsäure, Homocystein, Pyrrol-2-Carbonsäure, Picolinsäure, Indol, Indol-3-Methylacetat, Indol-3-Carbonsäure, Indolessigsäure, Purin, Azelainsäure, Nonansäure, Dopa, Dopamin, Melibiose, Fumarsäure, Paracetamol, p-Aminohippursäure, p-Kresolsulfat, symmetrisches Dimethylarginin, p-Hydroxymandelsäure, p-Hydroxyphenylessigsäure, Homogentisinsäure, Adipinsäure, Pimelinsäure, Isobuttersäure, Isoleucin, Isovaleriansäure, Zitronensäure, Isoursodeoxycholsäure, Isohyodeoxycholsäure, Isolithocholsäure, Isocholsäure, Isodesoxycholsäure, Glutarsäure, Glutaconsäure, Valeriansäure, Mandelsäure, Laurinsäure, Fructose-6-Phosphat, Citraconsäure, Zitronensäure, Citramalsäure, Ribonolacton, Ribonsäure, Raffinose, Palmitoleinsäure, Palmitinsäure, Norvalin, n-Hydroxyphenylessigsäure, Oxidiertes Glutathion, Aminoadipinsäure, Aminocapronsäure, Salicylsäure, Oleinsäure, Trehalose, Nikotinsäure, Pyroglutaminsäure, Ursocholsäure, Ursodeoxycholsäure, Tauro-a-muricholsäure, Tauro-b-muricholsäure, Tauro-w-muricholsäure, Tauroursodeoxycholsäure, Taurohyocholsäure, Taurohyodeoxycholsäure, Taurolithocholsäure, Taurocholsäure, Taurodehydrocholsäure, Taurochenodeoxycholsäure, Hyocholsäure, Hyodeoxycholsäure, Succinylaceton, Bernsteinsäure, Citrullin, Glycodehydrocholsäure, Glykolithocholsäure, Glykodeoxycholsäure, Glycin, Glykocheno-desoxycholsäure, Glyceraldehyd, Glyceraldehyd-3-Phosphat, Cholin-Glycerophosphat, Glycoursodesoxycholsäure, Glykohyocholsäure, Glykohyodesoxycholsäure, Glykocholsäure, Glyprolin, Glycyl-L-Leucin, Mannose-6-Phosphorsäure, Mannitol, Methylmalonsäure, Methylbernsteinsäure, Ameisensäure, Caprinsäure, Lithocholsäure, Selenomethionin, Thiamin, Glykolithocholsulfat, Stearinsäure, Liothyronin, Dihydroxyacetonphosphat, Phosphoribosylpyrophosphat, Kreatinphosphat, Hydroxypyruvinsäure, Glycinhydroxyphenylacetat, Zimtsäure, Sarcosin, Carnosin, Kreatin, Inositol, Adrenalin, Cholin, Cholsäure, Dehydrocholsäure, Demethylcholsäure, Adenosinmonophosphat, Arachidonsäure, Phenylbuttersäure, Phenethylamin, Phenylpropionsäure, Phenylmilchsäure, Benzamid, Benzoesäure, Oxalsäure, Shikimisäure, Glucarsäure, Glucose-6-Phosphat, Glucoselacton, Sebacinsäure, Ricinolsäure, Saccharose, Methioninsulfoxid, Itaconsäure, Melatonin, Glutathion, Myristinsäure, Erythronsäure, Erythrose, Suberinsäure, Caprylsäure, Phthalsäure, Weinsäure, Tyramin, Chinasäure, m-Aminobenzoesäure, asymmetrisches Dimethylarginin, Gerbsäure, cis-10,12-Octadecadiensäure, cis-12,15-Heneicosadiensäure, cis-12-Tridecensäure, cis-15-Tetracosensäure, cis-2-Hydroxyzimtsäure, cis-9-Tetradecensäure, cis-10-Heptadecensäure, cis-11-Dodecensäure, cis-4-Hydroxyprolin, cis-5-Dodecensäure, cis-7-Hexadecensäure, cis-9-Heptadecensäure, cis-Aconitsäure, Vanillinsäure, Hippursäure, Maleinsäure, Homovanillinsäure, Ornithin, Guanosinmonophosphat, Guanosintriphosphat, Anserin, Chenodesoxycholsäure, Maltotriose, Maltitol, Rhamnose und Murideoxycholsäure.

11. Verwendung des Stoffwechselchips nach einem der Ansprüche 8 bis 10, umfassend die folgenden Schritte:
a) Sammeln einer biologischen Probe;
b) je nach Typ der Probe, Herstellen eines entsprechenden Extrakts der biologischen Probe unter Verwendung des entsprechenden Verfahrens nach Anspruch 3 oder 4 oder 5;
c) Zugeben der hergestellten Extrakte der biologischen Proben in gleichen Mengen in jedem Well des Stoffwechselchips, Zugeben des gleichen Volumens einer 3-Nitrophenylhydrazin-Methanol-Lösung und einer 1-(3-Dimethylaminopropyl)-3-Ethylcarbodiimid-Pyridin-Lösung in jedem Well, und Durchführen eines gleichmäßigen Wirbelmischens und Erwärmens zur Derivatisierung, wobei die Konzentration an verwendetem 3-Nitrophenylhydrazin 100-320 mmol/L, die Konzentration an 1-(3-Dimethylaminopropyl)-3-Ethylcarbodiimid 50-200 mmol/L, die Reaktionstemperatur 20-60°C, und die Reaktionszeit 10-120 Minuten beträgt;
d) Zugeben einer mit Kohlenstoff-13-markierten Isotopen-Interne-Standard-Lösung, die aus der Reaktion von 3-Nitrophenylhydrazin und 1-(3-Dimethylaminopropyl)-3-ethylcarbodiimid erhalten wurde, zu dem in Schritt c) erhaltenen derivatisierten Extrakt der biologischen Probe; und
e) Zugeben einer Methanol-Wasser-Mischlösung in jedem Well in dem Stoffwechselchip in Schritt d) zur Verdünnung, Platzieren des Stoffwechselchips in einem Tandem-Massenspektrometer zur Bestimmung von Aminosäure, Phenol-, Phenyl- oder Benzylderivat, Indol, organischer Säure, Fettsäure, Zucker und Gallensäure durch Flüssigchromatographie-Massenspektrometrie und Berechnen von Konzentrationen von Zielmetaboliten in der Probe anhand der Ergebnisse.

## Revendications

1. Procédé de détection quantitative de multiples composantes métaboliques dans un échantillon biologique, comprenant la réalisation d'un traitement de dérivation sur l'échantillon biologique, et puis, la détection de l'échantillon biologique dérivé par chromatographie en phase liquide-spectrométrie de masse, dans lequel, pendant le traitement de dérivation, de la 3-nitrophénylhydrazine est utilisée comme réactif de dérivation et du 1-(3-diméthylaminopropyl)-3-éthylcarbodiimide est utilisé comme catalyseur de réaction de dérivation ; l'échantillon biologique est choisi parmi des échantillons d'urine, de sang, de liquide céphalorachidien, de tissus, de cellules, de salive et de matières fécales d'un mammifère ; les multiples composantes métaboliques dans l'échantillon biologique sont choisies parmi l'un ou plusieurs parmi un acide aminé, un dérivé phénolique, phénylique ou benzylique, un indole, un acide organique, un acide gras, un sucre et un acide biliaire, et ont des teneurs différentes ; dans lequel le procédé comprend les étapes suivantes consistant à :
a) prélever un échantillon biologique ;
b) extraire l'échantillon biologique à l'aide d'un solvant mixte composé de méthanol, de chloroforme et d'eau, effectuer une centrifugation, puis prélever un surnageant, à savoir un extrait d'échantillon biologique ;
c) ajouter le même volume d'une solution de 3-nitrophénylhydrazine dans le méthanol et d'une solution de 1-(3-diméthylaminopropyl)-3-éthylcarbodiimide dans la pyridine à l'extrait d'échantillon biologique obtenu à b), et effectuer un mélange uniforme avec vortex et un chauffage pour la dérivation, dans lequel la concentration de 3-nitrophénylhydrazine utilisée est de 100 à 320 mmol/L, la concentration de 1-(3-diméthylaminopropyl)-3-éthylcarbodiimide est de 50 à 200 mmol/L, la température de réaction est de 20 à 60 °C, et la durée de réaction est de 10 à 120 minutes ;
d) ajouter une solution standard interne d'isotope marqué au carbone 13 obtenue à partir de la réaction de 3-nitrophénylhydrazine et de 1-(3-diméthylaminopropyl)-3-éthylcarbodiimide à l'extrait d'échantillon biologique dérivé obtenu à c) ; et
e) ajouter une solution mixte méthanol-eau à l'échantillon à d) pour la dilution, et déterminer un acide aminé, un dérivé phénolique, phénylique ou benzylique, un indole, un acide organique, un acide gras, un sucre et un acide biliaire par chromatographie en phase liquide-spectrométrie de masse.

2. Procédé de détection selon la revendication 1, dans lequel, à l'étape c), la concentration de 3-nitrophénylhydrazine utilisée est de 150 à 220 mmol/L, la concentration de 1-(3-diméthylaminopropyl)-3-éthylcarbodiimide est de 80 à 120 mmol/L, la température de réaction est de 20 à 40 °C, et la durée de réaction est de 30 à 60 minutes.

3. Procédé de détection selon la revendication 1, dans lequel, lorsque l'échantillon biologique est de l'urine, du sang, de la salive ou du liquide céphalorachidien, un procédé de traitement de l'échantillon biologique à l'étape b) comprend : le prélèvement d'une quantité appropriée de l'échantillon biologique, l'extraction de l'échantillon biologique à l'aide d'un solvant mixte de méthanol froid, de chloroforme et d'eau dans un rapport volumique de 3:1:1, l'agitation du mélange pendant quelques secondes, la réalisation de la centrifugation de l'échantillon à une vitesse de rotation de 10 000 à 20 000 tr/min à basse température pendant 5 à 15 minutes, et le transfert du surnageant dans un flacon en verre d'échantillonneur automatique pour un traitement de dérivation ultérieur.

4. Procédé de détection selon la revendication 1, dans lequel, lorsque l'échantillon biologique est un échantillon de matières fécales, un procédé de traitement de l'échantillon biologique à l'étape b) comprend : la lyophilisation de l'échantillon de matières fécales ; l'homogénéisation d'une quantité appropriée de l'échantillon de matières fécales lyophilisé à l'aide d'une quantité appropriée d'un solvant mixte de méthanol froid, de chloroforme et d'eau dans un rapport volumique de 3:1:1, la réalisation de la centrifugation de l'échantillon à une vitesse de rotation de 10 000 à 20 000 tr/min à basse température pendant 15 à 30 minutes, et le transfert du surnageant dans un flacon en verre d'échantillonneur automatique pour un traitement de dérivation ultérieur.

5. Procédé de détection selon la revendication 1, dans lequel, lorsque l'échantillon biologique est un échantillon de tissus ou de cellules, un procédé de traitement de l'échantillon biologique à l'étape b) comprend : l'ajout d'une quantité appropriée d'un solvant mixte composé de méthanol froid, de chloroforme et d'eau dans un rapport volumique de 3:1:1 dans l'échantillon pour l'homogénéisation, la réalisation de la centrifugation de l'échantillon à une vitesse de rotation de 10 000 à 20 000 tr/min à 4 °C pendant 15 à 30 minutes, et le transfert du surnageant dans un flacon en verre d'échantillonneur automatique pour un traitement de dérivation ultérieur.

6. Procédé de détection selon la revendication 1, dans lequel le rapport volumique entre du méthanol et de l'eau à l'étape e) est de 1:1.

7. Procédé de détection selon l'une quelconque des revendications 1 à 6, dans lequel les multiples composantes métaboliques de l'échantillon biologique peuvent être choisies parmi les multiples métabolites suivants : fructose 1,6-diphosphate, acide 10Z-nonadécénoïque, acide trans-11-octadécénoïque, acide cis-11-octadécénoïque, acide 12-déhydrocholique, acide 12-hydroxystéarique, acide 12-cétolithocholique, 1-méthylhistidine, acide 2,2-diméthylsuccinique, acide 2,3-diaminopropionique, glucoside acide 24-chénodésoxycholique, acide 2-buténoïque, acide 2-oxoadipique, acide 2-méthyl-4-penténoïque, 2-méthyl-β-alanine, acide 2-méthylbutyrique, acide 2-méthylhexanoïque, acide 2-méthylglutarique, acide 2-méthylglutamique, acide 2-furoïque, acide 2-hydroxy-2-méthylbutyrique, acide 2-hydroxy-3-méthylbutyrique, acide 2-hydroxybutyrique, acide 2-hydroxycaproïque, acide 2-hydroxycinnamique, acide 2-hydroxyphénylacétique, acide 2-hydroxyhippurique, acide 2-phénylpropionique, 2-phénylglycine, acide 3-(3-hydroxyphényl)-3-hydroxypropionique, acide 3-(4-hydroxyphényl)lactique, acide 3,4-dihydroxymandélique, acide 3,4-dihydroxyphénylpropionique, 3,4 -déhydro-DL-proline, 3,5-diiodo-L-thyroxine, acide 3β-cholique, acide 3-pyridineacétique, acide 3-indoleacrylique, acide 3-indolepropionique, 3-indoleacétamide, 3-oxyalanine, acide 3-aminosalicylique, 3-chloro-L-tyrosine, acide 3-méthyl-2-oxobutyrique, acide 3-méthyl-2-oxovalérique, 3-méthylindole, acide 3-méthyladipique, acide 3-méthylglutamique, 3-nitrotyrosine, sulfate d'acide 3-taurolithocholique, sulfate d'acide 3-lithocholique, acide 3-hydroxy-2-aminobenzoïque, acide 3-hydroxybutyrique, acide 3-hydroxypropionique, acide 3-hydroxyisovalérique, acide 3-hydroxyphénylacétique, acide 3-hydroxyhippurique, acide 3-déhydrocholique, acide 3-phénylbutyrique, acide 4-méthyl-2-oxovalérique, acide 4-méthylhexanoïque, acide 4-méthoxyphénylacétique, acide 4-hydroxy-3-méthoxymandélique, acide 4-hydroxycinnamique, acide 4-hydroxybenzoïque, acide 4-hydroxyhippurique, acide 4-hydroxyphényllactique, acide 4-hydroxyphénylpyruvique, acide 4-phénylbutyrique, acide 5-aminolévulinique, 5-hydroxytryptophane, 5-hydroxytryptamine, 5-hydroxylysine, acide 6,7-dicétolithocholique, acide 6-phosphogluconique, acide 6-cétolithocholique, acide 7,12-dicétolithocholique, acide 7-cétolithocholique, acide 7-cétodésoxycholique, acide linoléique 9,11-conjugué, acide D-2-hydroxyglutarique, D-galactose, D-xylose, D-xylulose, D-fructose, D-ribose, D-ribose-5-phosphate, D-ribulose, D-ribulose-5-phosphate, D-mannose, D-glucose, D-maltose, acide L-2-aminobutyrique, acide L-3-phényllactique, L-alanine, L-sérine, L-acétylcarnitine, acide L-lactique, L-allothréonine, L-cystéine, L-homosérine, L-homocystéine, L-homocitrulline, acide L-pipécolique, acide L-aspartique, L-asparagine, L-sorbose, acide L-lignique, L-norleucine, L-kynurénine, L-thyronine, L-arginine, L-histidine, L-valine, L-cystine, L-cystathionine, L-proline, L-tryptophane, L-thréonine, L-phénylalanine, acide L-malique, L-méthionine, L-glutamine, acide L-glutamique, L-lysine, L-tyrosine, L-arabinose, N-(3-phénylpropionyl)glycine, N-acétyl-L-alanine, N-acétyl-L-acide aspartique, N-acétyl-L-phénylalanine, N-acétyl-L-méthionine, N-acétyl-L-tyrosine, N-acétylserine, N-acétyl-D-glucosamine, N-acétyl-L-phénylalanine, N-acétylmannosamine, acide N-acétylneuraminique, N-acétylhistidine, N-acétylhydroxytryptamine, N-acétyltryptophane, N-acétylglutamine, N-acétyllysine, N-acétylornithine, N-méthylnicotinamide, N-phénylacétyl-glutamine, N-phénylacétylphénylalanine, S-adénosine homocystéine, α-D-glucose, α-lactose, acide α-linolénique, acide α-hydroxyisobutyrique, acide α-cétoglutarique, acide α-muricholique, β-D-tréhalose, β-alanine, acide β-ursocholique, acide β-muricholique, γ-L-glutamyl-L-alanine, acide γ-linolénique, acide γ-aminobutyrique, acide ω-muricholique, acide butyrique, nitroxyde de triméthylamine, adénosine triphosphate, acide malonique, acide propionique, acide acétoacétique, acétylcystéine, acétylglycine, acétylorthine, acide acétique, acétate de guanidine, acide glycolique, lactulose, lactoylglutathion, acide hénicosanoïque, acide cis-12-hénicosénoïque, acide heptacosanoïque, acide tricosanoïque, acide cis-14-tricosénoïque, acide docosanoïque, acide docosatriénoïque, acide cis-13,16-docosadiénoïque, acide docosapentaénoïque, acide docosahexaénoïque, acide docosatétraénoïque, acide trans-13-docosaénoïque, acide cis-13-docosaénoïque, acide pentacosanoïque, acide octacosanoïque, acide hexacosanoïque, acide tétracosanoïque, acide eicosanoïque, acide eicosatriénoïque, acide eicosadiénoïque, acide eicosapentaénoïque, acide trans-11-eicosénoïque, acide cis-11-eicosénoïque, acide cis-5-eicosénoïque, acide cis-8-eicosénoïque, diméthylglycine, adénosine diphosphate, acide linoléique, leucine, alloisoleucine, acide allolithocholique, acide allocholique, acide undécénoïque, acide heptadécanoïque, acide tridécanoïque, acide nonadécanoïque, acide nonadécadiénoïque, acide pentadécanoïque, acide galactonique, galactitol, mécystéine, acide protocatéchique, acide apocholique, acide déshydrolithocholique, acide nordésoxycholique, acide trans-9-tétradécénoïque, acide trans-aconitique, trans-4-hydroxyproline, acide trans-9-heptadécénoïque, acide trans-9-pentadécénoïque, acide trans-9-hexadécénoïque, acide trans-linolénique, acide trans-cinnamique, acide élaïdique, homocystéine, acide pyrrole-2-carboxylique, acide picolinique, indole, acétate d'indole-3-méthyle, acide indole-3-carboxylique, acide indoleacétique, purine, acide azélaïque, acide nonanoïque, dopa, dopamine, mélibiose, acide fumarique, acétaminophène, acide p-aminohippurique, sulfate de p-crésol, diméthylarginine symétrique, acide p-hydroxymandélique, acide p-hydroxyphénylacétique, acide homogentisique, acide adipique, acide pimélique, acide isobutyrique, isoleucine, acide isovalérique, acide citrique, acide isoursodésoxycholique, acide isohyodésoxycholique, acide isolithocholique, acide isocholique, acide isodésoxycholique, acide glutarique, acide glutaconique, acide valérique, acide mandélique, acide laurique, fructose-6-phosphate, acide citraconique, acide citrique, acide citramalique, ribonolactone, acide ribonique, raffinose, acide palmitoléique, acide palmitique, norvaline, acide n-hydroxyphénylacétique, glutathion oxydé, acide aminoadipique, acide aminocaproïque, acide salicylurique, acide oléique, tréhalose, acide nicotinique, acide pyroglutamique, acide ursocholique, acide ursodésoxycholique, acide tauro-a-muricholique, acide tauro-b-muricholique, acide tauro-w-muricholique, acide tauroursodésoxycholique, acide taurohyocholique, acide taurohyodésoxycholique, acide taurolithocholique, acide taurocholique, acide taurodehydrocholique, acide taurochenodésoxycholique, acide hyocholique, acide hyodésoxycholique, succinylacétone, acide succinique, citrulline, acide glycodehydrocholique, acide glycolithocholique, acide glycodeoxycholique, glycine, acide glycochenodésoxycholique, glycéraldéhyde, glycéraldéhyde-3-phosphate, glycérophosphate de choline, acide glycoursodésoxycholique, acide glycohyocholique, acide glycohyodésoxycholique, acide glycocholique, glyproline, glycyl-L-leucine, acide mannose-6-phosphorique, mannitol, acide méthylmalonique, acide méthylsuccinique, acide formique, acide caprique, acide lithocholique, sélénométhionine, thiamine, sulfate de glycolithocholique, acide stéarique, liothyronine, phosphate de dihydroxyacétone, phosphoribosyl pyrophosphate, phosphate de créatine, acide hydroxypyruvique, hydroxyphénylacétate de glycine, acide cinnamique, sarcosine, carnosine, créatine, inositol, épinéphrine, choline, acide cholique, acide déhydrocholique, acide déméthylcholique, adénosine monophosphate, acide arachidonique, acide phénylpyruvique, phénéthylamine, acide phénylpropionique, acide phényllactique, benzamide, acide benzoïque, acide oxalique, acide shikimique, acide glucarique, glucose-6-phosphate, glucose lactone, acide sébacique, acide ricinoléique, saccharose, méthionine sulfoxyde, acide itaconique, mélatonine, glutathion, acide myristique, acide érythronique, érythrose, acide subérique, acide caprylique, acide phtalique, acide tartrique, tyramine, acide quinique, acide m-aminobenzoïque, diméthylarginine asymétrique, acide tannique, acide cis-10,12-octadécadiénoïque, acide cis-12,15-hénicosadiénoïque, acide cis-12-tridécénoïque, acide cis-15-tétracosénique, acide cis-2-hydroxycinnamique, acide cis-9-tétradécénoïque, acide cis-10-heptadécénoïque, acide cis-11-dodécénoïque, cis-4-hydroxyproline, acide cis-5-dodécénoïque, acide cis-7-hexadécénoïque, acide cis-9-heptadécénoïque, acide cis-aconitique, acide vanillique, acide hippurique, acide maléique, acide homovanillique, ornithine, guanosine monophosphate, guanosine triphosphate, ansérine, acide chénodésoxycholique, maltotriose, maltitol, rhamnose et acide muridésoxycholique.

8. Utilisation d'une puce métabolique dans le procédé de détection selon l'une quelconque des revendications 1 à 7, la puce métabolique comprenant un support de puce, un dispositif de filtration et une poudre solide sèche d'un produit standard et d'un produit de contrôle qualité, dans laquelle le support de puce est une plaque de microtitration, chaque puits de la plaque de microtitration est pourvu d'un dispositif de filtration indépendant, et la poudre obtenue par déshydratation ou lyophilisation de solutions du produit standard et du produit de contrôle qualité est placée sur le dispositif de filtration dans chaque puits de la plaque de microtitration ; le produit standard est choisi parmi l'un ou plusieurs parmi des produits standards à base d'acide aminé, de dérivé phénolique, phénylique ou benzylique, d'indole, d'acide organique, d'acide gras, de sucre et d'acide biliaire ; le produit de contrôle qualité est choisi parmi l'un ou plusieurs parmi des produits standards à base d'acide aminé, de dérivé phénolique, phénylique ou benzylique, d'indole, d'acide organique, d'acide gras, de sucre et d'acide biliaire, correspondant aux produits standards ci-dessus.

9. Utilisation de la puce métabolique selon la revendication 8, dans laquelle la plaque de microtitration est choisie parmi une plaque à 48 puits, une plaque à 96 puits et une plaque à 384 puits, et le dispositif de filtration est une membrane filtrante fabriquée de polyfluorure de vinylidène, d'acétate de cellulose ou de nylon, ayant une taille de pores comprise entre 0,20 et 0,45 micron.

10. Utilisation de la puce métabolique selon la revendication 8, dans laquelle le produit standard et le produit de contrôle qualité peuvent être choisis parmi les multiples métabolites suivants : fructose 1,6-diphosphate, acide 10Z-nonadécénoïque, acide trans-11-octadécénoïque, acide cis-11-octadécénoïque, acide 12-déhydrocholique, acide 12-hydroxystéarique, acide 12-cétolithocholique, 1-méthylhistidine, acide 2,2-diméthylsuccinique, acide 2,3-diaminopropionique, glucoside acide 24-chénodésoxycholique, acide 2-buténoïque, acide 2-oxoadipique, acide 2-méthyl-4-penténoïque, 2-méthyl-β-alanine, acide 2-méthylbutyrique, acide 2-méthylhexanoïque, acide 2-méthylglutarique, acide 2-méthylglutamique, acide 2-furoïque, acide 2-hydroxy-2-méthylbutyrique, acide 2-hydroxy-3-méthylbutyrique, acide 2-hydroxybutyrique, acide 2-hydroxycaproïque, acide 2-hydroxycinnamique, acide 2-hydroxyphénylacétique, acide 2-hydroxyhippurique, acide 2-phénylpropionique, 2-phénylglycine, acide 3-(3-hydroxyphényl)-3-hydroxypropionique, acide 3-(4-hydroxyphényl)lactique, acide 3,4-dihydroxymandélique, acide 3,4-dihydroxyphénylpropionique, 3,4 -déhydro-DL-proline, 3,5-diiodo-L-thyroxine, acide 3β-cholique, acide 3-pyridineacétique, acide 3-indoleacrylique, acide 3-indolepropionique, 3-indoleacétamide, 3-oxyalanine, acide 3-aminosalicylique, 3-chloro-L-tyrosine, acide 3-méthyl-2-oxobutyrique, acide 3-méthyl-2-oxovalérique, 3-méthylindole, acide 3-méthyladipique, acide 3-méthylglutamique, 3-nitrotyrosine, sulfate d'acide 3-taurolithocholique, sulfate d'acide 3-lithocholique, acide 3-hydroxy-2-aminobenzoïque, acide 3-hydroxybutyrique, acide 3-hydroxypropionique, acide 3-hydroxyisovalérique, acide 3-hydroxyphénylacétique, acide 3-hydroxyhippurique, acide 3-déhydrocholique, acide 3-phénylbutyrique, acide 4-méthyl-2-oxyvalérique, acide 4-méthylhexanoïque, acide 4-méthoxyphénylacétique, acide 4-hydroxy-3-méthoxymandélique, acide 4-hydroxycinnamique, acide 4-hydroxybenzoïque, acide 4-hydroxyhippurique, acide 4-hydroxyphényllactique, acide 4-hydroxyphénylpyruvique, acide 4-phénylbutyrique, acide 5-aminolévulinique, 5-hydroxytryptophane, 5-hydroxytryptamine, 5-hydroxylysine, acide 6,7-dicétolithocholique, acide 6-phosphogluconique, acide 6-cétolithocholique, acide 7,12-dicétolithocholique, acide 7-cétolithocholique, acide 7-cétodésoxycholique, acide linoléique 9,11-conjugué, acide D-2-hydroxyglutarique, D-galactose, D-xylose, D-xylulose, D-fructose, D-ribose, D-ribose-5-phosphate, D-ribulose, D-ribulose-5-phosphate, D-mannose, D-glucose, D-maltose, acide L-2-aminobutyrique, acide L-3-phényllactique, L-alanine, L-sérine, L-acétylcarnitine, acide L-lactique, L-allothréonine, L-cystéine, L-homosérine, L-homocystéine, L-homocitrulline, acide L-pipécolique, acide L-aspartique, L-asparagine, L-sorbose, acide L-lignique, L-norleucine, L-kynurénine, L-thyronine, L-arginine, L-histidine, L-valine, L-cystine, L-cystathionine, L-proline, L-tryptophane, L-thréonine, L-phénylalanine, acide L-malique, L-méthionine, L-glutamine, acide L-glutamique, L-lysine, L-tyrosine, L-arabinose, N-(3-phénylpropionyl)glycine, N-acétyl-L-alanine, N-acétyl-L-acide aspartique, N-acétyl-L-phénylalanine, N-acétyl-L-méthionine, N-acétyl-L-tyrosine, N-acétylserine, N-acétyl-D-glucosamine, N-acétyl-L-phénylalanine, N-acétylmannosamine, acide N-acétylneuraminique, N-acétylhistidine, N-acétylhydroxytryptamine, N-acétyltryptophane, N-acétylglutamine, N-acétyllysine, N-acétylornithine, N-méthylnicotinamide, N-phénylacétyl-glutamine, N-phénylacétylphénylalanine, S-adénosine homocystéine, α-D-glucose, α-lactose, acide α-linolénique, acide α-hydroxyisobutyrique, acide α-cétoglutarique, acide α-muricholique, β-D-tréhalose, β-alanine, acide β-ursocholique, acide β-muricholique, γ-L-glutamyl-L-alanine, acide γ-linolénique, acide γ-aminobutyrique, acide ω-muricholique, acide butyrique, nitroxydes de triméthylamine, adénosine triphosphate, acide malonique, acide propionique, acide acétoacétique, acétylcystéine, acétylglycine, acétylorthine, acide acétique, acétate de guanidine, acide glycolique, lactulose, lactoylglutathion, acide hénicosanoïque, acide cis-12-hénicosénoïque, acide heptacosanoïque, acide tricosanoïque, acide cis-14-tricosénoïque, acide docosanoïque, acide docosatriénoïque, acide cis-13,16-docosadiénoïque, acide docosapentaénoïque, acide docosahexaénoïque, acide docosatétraénoïque, acide trans-13-docosaénoïque, acide cis-13-docosaénoïque, acide pentacosanoïque, acide octacosanoïque, acide hexacosanoïque, acide tétracosanoïque, acide eicosanoïque, acide eicosatriénoïque, acide eicosadiénoïque, acide eicosapentaénoïque, acide trans-11-eicosénoïque, acide cis-11-eicosénoïque, acide cis-5-eicosénoïque, acide cis-8-eicosénoïque, diméthylglycine, adénosine diphosphate, acide linoléique, leucine, alloisoleucine, acide allolithocholique, acide allocholique, acide undécénoïque, acide heptadécanoïque, acide tridécanoïque, acide nonadécanoïque, acide nonadécadiénoïque, acide pentadécanoïque, acide galactonique, galactitol, mécystéine, acide protocatéchique, acide apocholique, acide déshydrolithocholique, acide nordésoxycholique, acide trans-9-tétradécénoïque, acide trans-aconitique, trans-4-hydroxyproline, acide trans-9-heptadécénoïque, acide trans-9-pentadécénoïque, acide trans-9-hexadécénoïque, acide trans-linolénique, acide trans-cinnamique, acide élaïdique, homocystéine, acide pyrrole-2-carboxylique, acide picolinique, indole, acétate d'indole-3-méthyle, acide indole-3-carboxylique, acide indoleacétique, purine, acide azélaïque, acide nonanoïque, dopa, dopamine, mélibiose, acide fumarique, acétaminophène, acide p-aminohippurique, sulfate de p-crésol, diméthylarginine symétrique, acide p-hydroxymandélique, acide p-hydroxyphénylacétique, acide homogentisique, acide adipique, acide pimélique, acide isobutyrique, isoleucine, acide isovalérique, acide citrique, acide isoursodésoxycholique, acide isohyodésoxycholique, acide isolithocholique, acide isocholique, acide isodésoxycholique, acide glutarique, acide glutaconique, acide valérique, acide mandélique, acide laurique, fructose-6-phosphate, acide citraconique, acide citrique, acide citramalique, ribonolactone, acide ribonique, raffinose, acide palmitoléique, acide palmitique, norvaline, acide n-hydroxyphénylacétique, glutathion oxydé, acide aminoadipique, acide aminocaproïque, acide salicylurique, acide oléique, tréhalose, acide nicotinique, acide pyroglutamique, acide ursocholique, acide ursodésoxycholique, acide tauro-a-muricholique, acide tauro-b-muricholique, acide tauro-w-muricholique, acide tauroursodésoxycholique, acide taurohyocholique, acide taurohyodésoxycholique, acide taurolithocholique, acide taurocholique, acide taurodehydrocholique, acide taurochenodésoxycholique, acide hyocholique, acide hyodésoxycholique, succinylacétone, acide succinique, citrulline, acide glycodehydrocholique, acide glycolithocholique, acide glycodeoxycholique, glycine, acide glycochenodésoxycholique, glycéraldéhyde, glycéraldéhyde-3-phosphate, glycérophosphate de choline, acide glycoursodésoxycholique, acide glycohyocholique, acide glycohyodésoxycholique, acide glycocholique, glyproline, glycyl-L-leucine, acide mannose-6-phosphorique, mannitol, acide méthylmalonique, acide méthylsuccinique, acide formique, acide caprique, acide lithocholique, sélénométhionine, thiamine, sulfate de glycolithocholique, acide stéarique, liothyronine, phosphate de dihydroxyacétone, phosphoribosyl pyrophosphate, phosphate de créatine, acide hydroxypyruvique, hydroxyphénylacétate de glycine, acide cinnamique, sarcosine, carnosine, créatine, inositol, épinéphrine, choline, acide cholique, acide déhydrocholique, acide déméthylcholique, adénosine monophosphate, acide arachidonique, acide phénylpyruvique, phénéthylamine, acide phénylpropionique, acide phényllactique, benzamide, acide benzoïque, acide oxalique, acide shikimique, acide glucarique, glucose-6-phosphate, glucose lactone, acide sébacique, acide ricinoléique, saccharose, méthionine sulfoxyde, acide itaconique, mélatonine, glutathion, acide myristique, acide érythronique, érythrose, acide subérique, acide caprylique, acide phtalique, acide tartrique, tyramine, acide quinique, acide m-aminobenzoïque, diméthylarginine asymétrique, acide tannique, acide cis-10,12-octadécadiénoïque, acide cis-12,15-hénicosadiénoïque, acide cis-12-tridécénoïque, acide cis-15-tétracosénique, acide cis-2-hydroxycinnamique, acide cis-9-tétradécénoïque, acide cis-10-heptadécénoïque, acide cis-11-dodécénoïque, cis-4-hydroxyproline, acide cis-5-dodécénoïque, acide cis-7-hexadécénoïque, acide cis-9-heptadécénoïque, acide cis-aconitique, acide vanillique, acide hippurique, acide maléique, acide homovanillique, ornithine, guanosine monophosphate, guanosine triphosphate, ansérine, acide chénodésoxycholique, maltotriose, maltitol, rhamnose et acide muridésoxycholique.

11. Utilisation de la puce métabolique selon l'une quelconque des revendications 8 à 10, comprenant les étapes suivantes consistant à :
a) prélever un échantillon biologique ;
b) en fonction du type d'échantillon, préparer un extrait d'échantillon biologique correspondant en utilisant le procédé correspondant selon la revendication 3 ou 4 ou 5 ;
c) ajouter l'extrait d'échantillon biologique préparé dans chaque puits de la puce métabolique en quantité égale, ajouter le même volume d'une solution de 3-nitrophénylhydrazine dans le méthanol et d'une solution de 1-(3-diméthylaminopropyl)-3-éthylcarbodiimide dans la pyridine à chaque puits, et effectuer un mélange uniforme avec vortex et un chauffage pour la dérivation, dans lequel la concentration de 3-nitrophénylhydrazine utilisée est de 100 à 320 mmol/L, la concentration de 1-(3-diméthylaminopropyl)-3-éthylcarbodiimide est de 50 à 200 mmol/L, la température de réaction est de 20 à 60 °C, et la durée de réaction est de 10 à 120 minutes ;
d) ajouter une solution standard interne d'isotope marqué au carbone 13 obtenue à partir de la réaction de 3-nitrophénylhydrazine et de 1-(3-diméthylaminopropyl)-3-éthylcarbodiimide à l'extrait d'échantillon biologique dérivé obtenu à c) ; et
e) ajouter une solution mixte méthanol-eau dans chaque puits de la puce métabolique à d) pour la dilution, placer la puce métabolique dans un spectromètre de masse en tandem pour déterminer un acide aminé, un dérivé phénolique, phénylique ou benzylique, un indole, un acide organique, un acide gras, un sucre et un acide biliaire par chromatographie en phase liquide-spectrométrie de masse, et calculer des concentrations de métabolites cibles dans l'échantillon sur la base des résultats.
